(19) Europäisches Patentamt
European Patent Office
Office européen des brevets

(11) Publication number: **0 415 776 A2**

(12) **EUROPEAN PATENT APPLICATION**

(21) Application number: 90309528.9

(22) Date of filing: 30.08.90

(51) Int. Cl.[5]: C07D 471/06, A61K 31/435, //(C07D471/06,221:00,209:00)

(30) Priority: 30.08.89 JP 223959/89

(43) Date of publication of application: 06.03.91 Bulletin 91/10

(84) Designated Contracting States: AT BE CH DE DK ES FR GB GR IT LI LU NL SE

(71) Applicant: Sankyo Company Limited
5-1 Nihonbashi Honcho 3-chome Chuo-ku
Tokyo(JP)

(72) Inventor: Hashimoto, Toshihiko, c/o Sankyo Company Limited
2-58, 1-chome, Hiromachi, Shinagawa-ku
Tokyo(JP)
Inventor: Fukazawa, Tetsuya, c/o Sankyo Company Limited
2-58, 1-chome, Hiromachi, Shinagawa-ku
Tokyo(JP)
Inventor: Masuko, Hidekazu, c/o Sankyo Company Limited
2-58, 1-chome, Hiromachi, Shinagawa-ku
Tokyo(JP)
Inventor: Shimoji, Yasuo, c/o Sankyo Company Limited
2-58, 1-chome, Hiromachi, Shinagawa-ku
Tokyo(JP)
Inventor: Koike, Hiroyuki, c/o Sankyo Company Limited
2-58, 1-chome, Hiromachi, Shinagawa-ku
Tokyo(JP)
Inventor: Mizuno, Hiroshi, c/o Sankyo Company Limited
2-58, 1-chome, Hiromachi, Shinagawa-ku
Tokyo(JP)

(74) Representative: Gibson, Christian John Robert et al
MARKS & CLERK 57/60 Lincoln's Inn Fields
London WC2A 3LS(GB)

# (54) Indolobenzoquinoline derivatives, their preparation and their use as anti-arrhythmic drugs.

(57) Optically active compounds of formula (I):

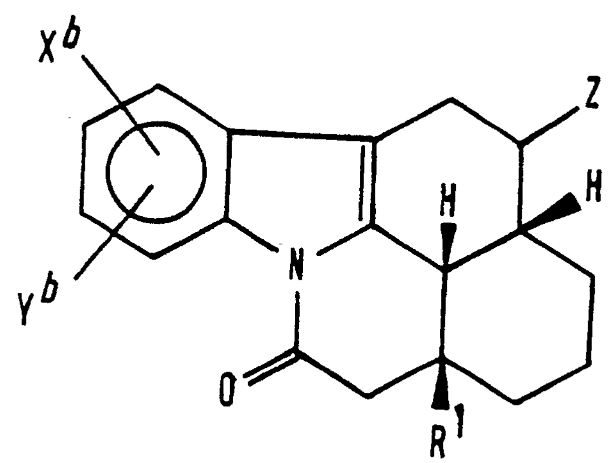

(I)

in which $R^1$ is hydrogen or alkyl; $X^b$ and $Y^b$ are hydrogen or hydroxy; and Z is $-NR^aR^b$ in which $R^a$ and $R^b$ are hydrogen, alkyl or hydroxyalkyl,



Xerox Copy Centre

or a cyclic amino group;
and pharmaceutically acceptable salts thereof have enhanced anti-arrhythmic activity and may be prepared by a stereospecific synthesis process.

# INDOLOBENZOQUINOLINE DERIVATIVES, THEIR PREPARATION AND THEIR USE AS ANTI-ARRHYTHMIC DRUGS

The present invention relates to a series of optically active indolobenzoquinoline derivatives which have excellent anti-arrhythmic activity; the invention also provides a stereospecific process for preparing the compounds, as well as methods and compositions using them.

It has long been known that certain condensed ring heterocyclic compounds, for example, the compound having the following formula (A):

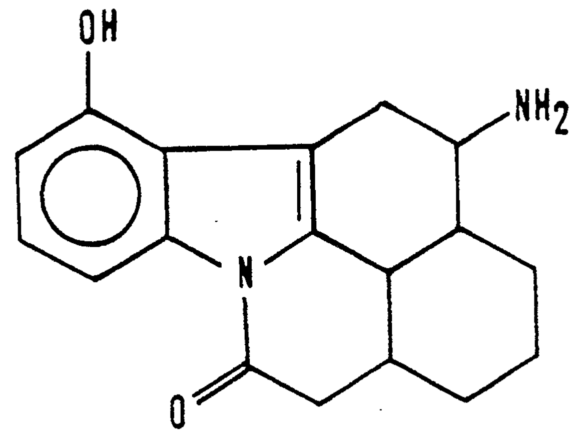

(A)

possess anti-arrhythmic and similar activities and have been found to be useful in therapy (see, for example, US A-4 716 162).

It is clear from this formula that these compounds can exist in the form of several optical isomers; however, in practice, they have always been produced as racemates.

We have now disoovered that the activities of the isomers are not the same and that one isomer is substantially more active than the other and, accordingly, is substantially more active than the racemic mixture. Accordingly, it would be desirable to resolve the isomers optically in order to obtain the optical isomer having the greater biological activity. However, optical resolution of these compounds has not yet been achieved. An alternative approach would be to develop a stereospecific process for preparing this optical isomer in a form having a high optical purity in a high yield and by means of simple operation.

We have now found that the optically active form of these condensed-ring heterocyclic compounds can be obtained in a form having a high optical purity and in high yield from an optically active starting material. Although the procedure is simple, the compounds can be obtained with a high optical purity and in high yields.

In accordance with the present invention, there are provided optically active compounds of formula (I):

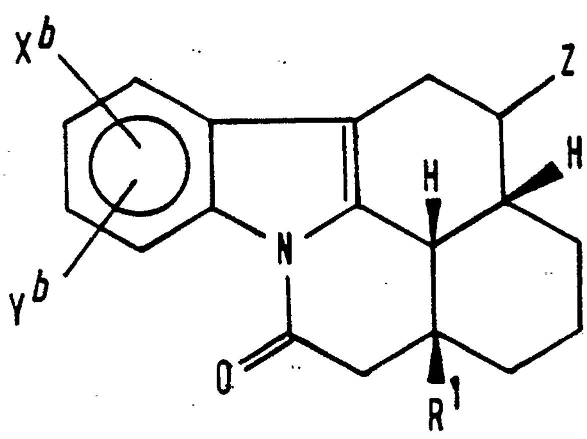

(I)

in which:

$R^1$ represents a hydrogen atom or an alkyl group having from 1 to 4 carbon atoms;
$X_b$ and $Y_b$ are the same or different and each represents a hydrogen atom or a hydroxy group; and
Z represents a group of formula $-NR^aR^b$
in which $R^a$ and $R^b$ are the same or different and each represents a hydrogen atom, an alkyl group having from 1 to 4 carbon atom or a hydroxyalkyl group having at least one hydroxy group and having from 1 to 4 carbon atoms,
or a cyclic amino group having from 3 to 6 ring atoms, of which 1 or 2 are nitrogen atoms, 0 or 1 is an oxygen or sulphur atom and the remainder is or are carbon atoms;
and pharmaceutically acceptable salts thereof.

The invention also provides a pharmaceutical composition for the treatment and prophylaxis of arrhythmia, which comprises an anti-arrhythmic compound in admixture with a pharmaceutically acceptable carrier or diluent, wherein the anti-arrhythmic compound is at least one optically active compound of formula (I) or a pharmaceutically acceptable salt thereof.

The invention still further provides the use of at least one optically active compound of formula (I) or a pharmaceutically acceptable salt thereof for the treatment or prophylaxis of arrhythmia in a mammal, whioh may be a human, and the use of these compounds for the manufacture of a medicament for such treatment or prophylaxis.

The invention also provides a process for preparing the optically active compounds of the present invention, which process comprises the steps:

(a) heating a compound of formula (IV):

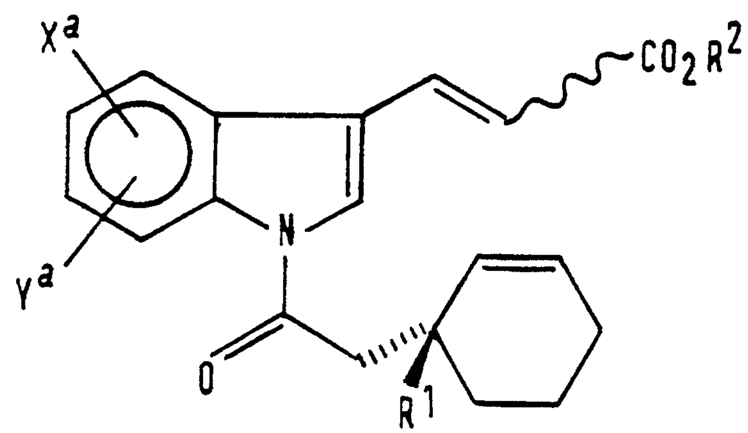

(IV)

(in which $R^1$ is as defined above; $R^2$ represents a carboxy-protecting group; and $X^a$ and $Y^a$ are the same or different and each represents a hydrogen atom or a protected hydroxy group) to afford a compound of formula (V):

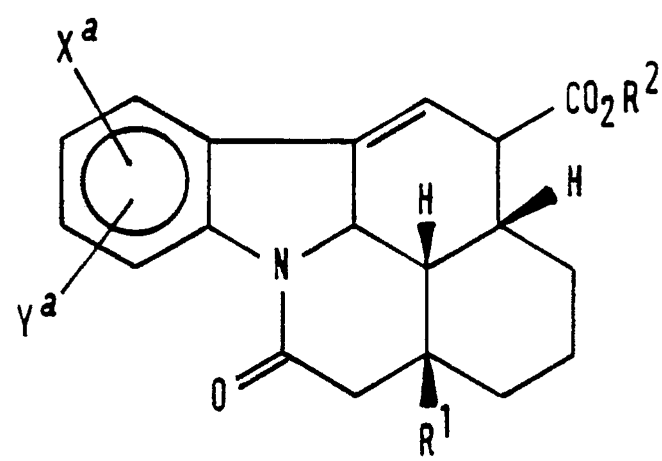

(V)

(in which $R^1$, $R^2$, $X^a$ and $Y^a$ are as defined above);

(b) isomerizing the compound of formula (V) and removing the carboxy-protecting group, to give a

compound of formula (VI):

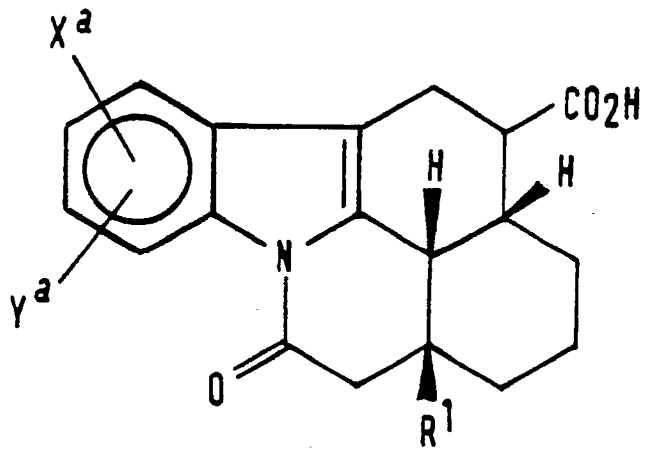

(VI)

(in which $R^1$, $X^a$ and $Y^a$ are as defined above);

(c) azidating and heating the compound of formula (VI), and then reacting it with an alcoholic compound of formula (IX):

$R^3OH$ (IX)

(in which $R^3$ represents an alkyl group having from 1 to 4 carbon atoms, an alkenyl group having from 3 to 6 carbon atoms or an aralkyl group having from 7 to 10 carbon atoms) to afford a carbamic acid ester compound of formula (VII):

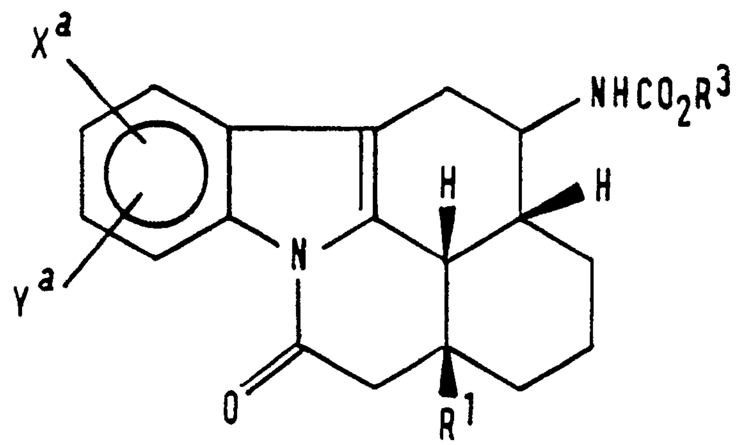

(VII)

(in which $R^1$, $R^3$, $X^a$ and $Y^a$ are as defined above);

(d) eliminating the group of formula $-CO_2R^3$ from said compound of formula (VII) to prepare the corresponding amino compound;

(e) reacting said amino compound with a compound of formula (X) or (XI):

$R^4$-Q (X)

or

Q-A-Q′ (XI)

(in which: $R^4$ represents an alkyl group having from 1 to 4 carbon atoms or an alkyl group having from 1 to 4 carbon atoms and having at least one hydroxy substituent; A represents an alkylene group having from 1 to 5 carbon atoms whose carbon chain is interrupted by 0 or 1 nitrogen, oxygen or sulphur atom; and Q and Q′ are the same or different and each represents a halogen atom); and

(f) removing the hydroxy-protecting groups;

(g) and, if desired, salifying the product.

Preferably the compound of formula (IV) is prepared by reacting an indole compound of formula (II):

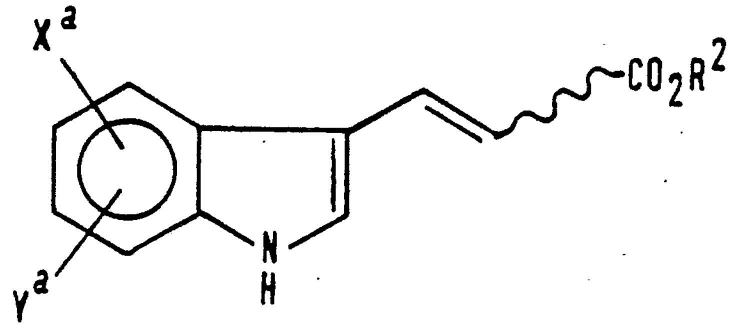

(II)

(in which $R^2$, $X^a$ and $Y^a$ are as defined above) or a reactive derivative thereof with an optically active cyclohexenylacetic acid of formula (III):

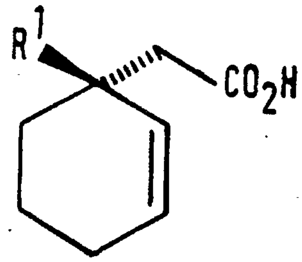

(III)

(in which $R^1$ is as defined above) or with a reactive derivative of said acid, to afford said compound of formula (IV).

In the accompanying drawings:

Figure 1 shows the course of ischemia-induced arrhythmias in male beagles with the passage of time after treatment with an optioally active compound of the present invention; and

Figure 2 shows similar results but in which the compound used was a racemate of the compound of the invention, demonstrating the superior activity of the optically active compound of the invention over the racemate.

For the avoidance of doubt, the peripheral numbering system employed herein for the naming of the compounds of the present invention is as shown on the following formula (B):

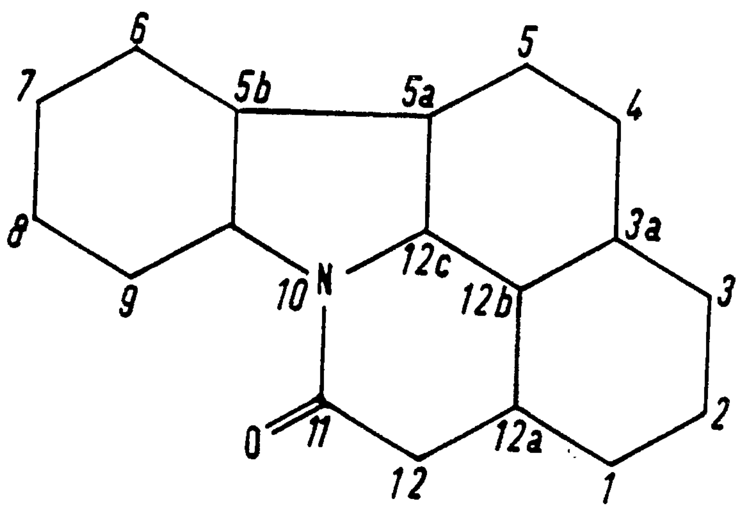

(B)

In the compounds of the invention, where $R^1$ represents an alkyl group, this has from 1 to 4 carbon atoms and may be a straight or branched chain group. Examples of such groups inolude the methyl, ethyl, propyl, isopropyl, butyl, isobutyl, sec-butyl and t-butyl groups, of which the methyl and ethyl groups are preferred.

Similarly, where $R^a$ and/or $R^b$ represents an alkyl group, this has from 1 to 4 carbon atoms and may be a straight or branched chain group. Examples of such groups include the methyl, ethyl, propyl, isopropyl, butyl, isobutyl, sec-butyl and t-butyl groups, of which the methyl and ethyl groups are preferred. Examples of hydroxyalkyl groups include the hydroxymethyl, 2-hydroxyethyl, 3-hydroxypropyl and 2-hydroxypropyl groups, of whioh the 2-hydroxyethyl group is preferred. In the group represented by Z, the two groups $R^a$ and $R^b$ may be the same or different. Preferably both are hydrogen atoms, both are alkyl groups, one is an alkyl group and the other is a hydrogen atom or both are hydroxyalkyl groups. Examples of groups which may be represented by Z include the amino, methylamino, ethylamino, propylamino, isopropylamino, butylamino, isobutylamino, dimethylamino, methylethylamino, diethylamino, methylpropylamino, methylbutylamino and bis(2-hydroxyethyl)amino groups, of whioh we prefer the ethylamino group, the dimethylamino group, the diethylamino group or the bis(2-hydroxyethyl)amino group.

Where Z represents a cyclic amino group, this has at least one nitrogen atom through which the group is attached to the remainder of the molecule. Additionally, it may optionally have a further one nitrogen atom or an oxygen or sulphur atom, preferably an oxygen or sulphur atom, in the heterocyclic ring; examples of such cyclic amino groups include the aziridinyl, azetidinyl, pyrrolidyl, piperidyl, piperazinyl, morpholinyl and thiomorpholinyl groups, of which we prefer the pyrrolidyl group or the piperidyl group.

Furthermore, the preferred configuration of the group represented by Z is the R- (^ -) configuration.

The compounds of the present invention contain a basic nitrogen atom and can, therefore form acid addition salts with suitable acids. There is, in principle, no restriction on the nature of the acids used to form such salts. However, where the resulting salt is intended for therapeutic use, it is necessary that the salt should be pharmaceutically acceptable, which, as is well known in the art, means that it should not have reduced activity (or unacceptably reduced activity) or increased toxicity (or unacceptably increased toxicity) as compared to the free base. Where, however, the salt is to be used for some other purpose, e.g. as an intermediate in the production of another, and possibly more active, compound, even this restriction does not apply. Examples of suitable acids include: inorganic acids, such as hydrochloric acid, hydrobromic acid, hydroiodic acid, sulphuric acid, nitric acid and phosphoric acid; organic carboxylic acids, such as formic acid, acetic acid, oxalic acid, malonic acid, maleic acid, fumaric acid, succinic acid, citric acid, tartaric acid, lactic acid, aspartic acid and benzoic acid; and organic sulphonic acids, such as methanesulphonic acid, ethanesulphonic acid, benzenesulphonic acid and p -toluenesulphonic acid.

The preferred classes of compounds of formula (I) are:

(A) those compounds in which $R^1$ represents a hydrogen atom, a methyl group or an ethyl group (particularly a hydrogen atom);

(B) those compounds in which one of $X^b$ and $Y^b$ represents a hydroxy group and the other represents a hydrogen atom;

(C) those oompounds in which Z represents an amino, ethylamino, dimethylamino, diethylamino, pyrrolidyl or piperidyl group (particularly an amino, ethylamino or dimethylamino group); and

(D) those compounds in whioh $X^b$ represents a hydroxy group at the 6- or 7- position (particularly at the 6-position) and $Y^b$ represents a hydrogen atom.

Specific examples of the compounds of the present invention are those compounds of formula (I) in whioh $R^1$, $X^b$, $Y^b$ and Z have the meanings shown in the following Table 1. In the Table, the abbreviation "Pyrd" refers to the 1-pyrrolidinyl group.

Table 1

| Cpd. No. | $R^1$ | $X^b$ | $Y^b$ | Z |
|---|---|---|---|---|
| 1-1 | H | 6-OH | H | $-NH_2$ |
| 1-2 | $CH_3$ | 6-OH | H | $-NH_2$ |
| 1-3 | $C_2H_5$ | 6-OH | H | $-NH_2$ |
| 1-4 | H | 7-OH | H | $-NH_2$ |
| 1-5 | H | 7-OH | 8-OH | $-NH_2$ |
| 1-6 | H | 6-OH | H | $-N(CH_3)_2$ |
| 1-7 | H | 6-OH | H | $-NHC_2H_5$ |
| 1-8 | H | 6-OH | H | Pyrd |
| 1-9 | H | 6-OH | H | $-N(CH_2CH_2OH)_2$ |
| 1-10 | H | 6-OH | H | $-N(C_2H_5)_2$ |
| 1-11 | H | 6-OH | H | $-NHCH_3$ |
| 1-12 | H | 6-OH | H | $-NH(CH_2CH_2OH)$ |
| 1-13 | $CH_3$ | 6-OH | H | $-N(CH_3)_2$ |
| 1-14 | $CH_3$ | 6-OH | H | $-NHC_2H_5$ |
| 1-15 | $CH_3$ | 6-OH | H | $-N(CH_2CH_2OH)_2$ |
| 1-16 | $C_2H_5$ | 6-OH | H | $-N(CH_3)_2$ |
| 1-17 | $C_2H_5$ | 6-OH | H | $-NHC_2H_5$ |
| 1-18 | $C_2H_5$ | 6-OH | H | $-N(CH_2CH_2OH)_2$ |

Of these, the most preferred compounds are Compounds No.:

1. (3a R ,4 R , 12a R , 12b S ) 4-Amino-2,3,3a,4,5,12,12a,12b-octahydro-6-hydroxyindolo[3,2,1,-ij]benz-[de]quinolin-11(1 H ) -one;

2. (3a R ,4 R , 12a R ,12b S ) 4-Amino-2,3,3a,4,5,12,12a,12b-octahydro-6-hydroxy-12a-methylindolo-[3,2,1,-ij]benz[de] quinolin-11(1 H )-one;

3. (3a R ,4 R ,12a R ,12b S ) 4-Amino-12a-ethyl-2,3,3a,4,5,12,12a,12b-octahydro-6-hydroxyindolo[3,2,1,-ij]benz[de]quinolin-11(1 H )-one;

6. (3a R , 4 R ,12a R ,12b S ) 4-(Dimethylamino-2,3,3a,4,5,12,12a,12b-octahydro-6-hydroxyindolo[3,2,1,-ij]benz[de]quinolin-11(1 H )-one;

7. (3a R ,4 R ,12a R ,12b S ) 4-Ethylamino-2,3,3a,4,5,12,12a,12b-octahydro-6-hydroxyindolo[3,2,1,-ij]-benz[de]quinolin-11 (1 H )-one;

8. (3a R ,4 R ,12a R ,12b S ) 2,3,3a,4,5,12,12a,12b-Octahydro-6-hydroxy-4-pyrrolidin-1-ylindolo-[3,2,1,-ij]-benz[de]-quinolin-11(1 H )-one; and

9. (3a R ,4 R , 12a R , 12b S ) 2,3,3a,4,5,12,12a,12b-Octahydro-6-hydroxy-4-bis(2-hydroxyethyl)-aminoindolo[3,2,1-ij]benz[de]quinolin-11( 1 H ) -one.

Also preferred are salts, especially hydrochlorides, of the above compounds.

The compounds of the present invention may be prepared by the general procedure outlined above; this is shown in greater detail by the following Reaction Scheme A:

Reaction Scheme: A

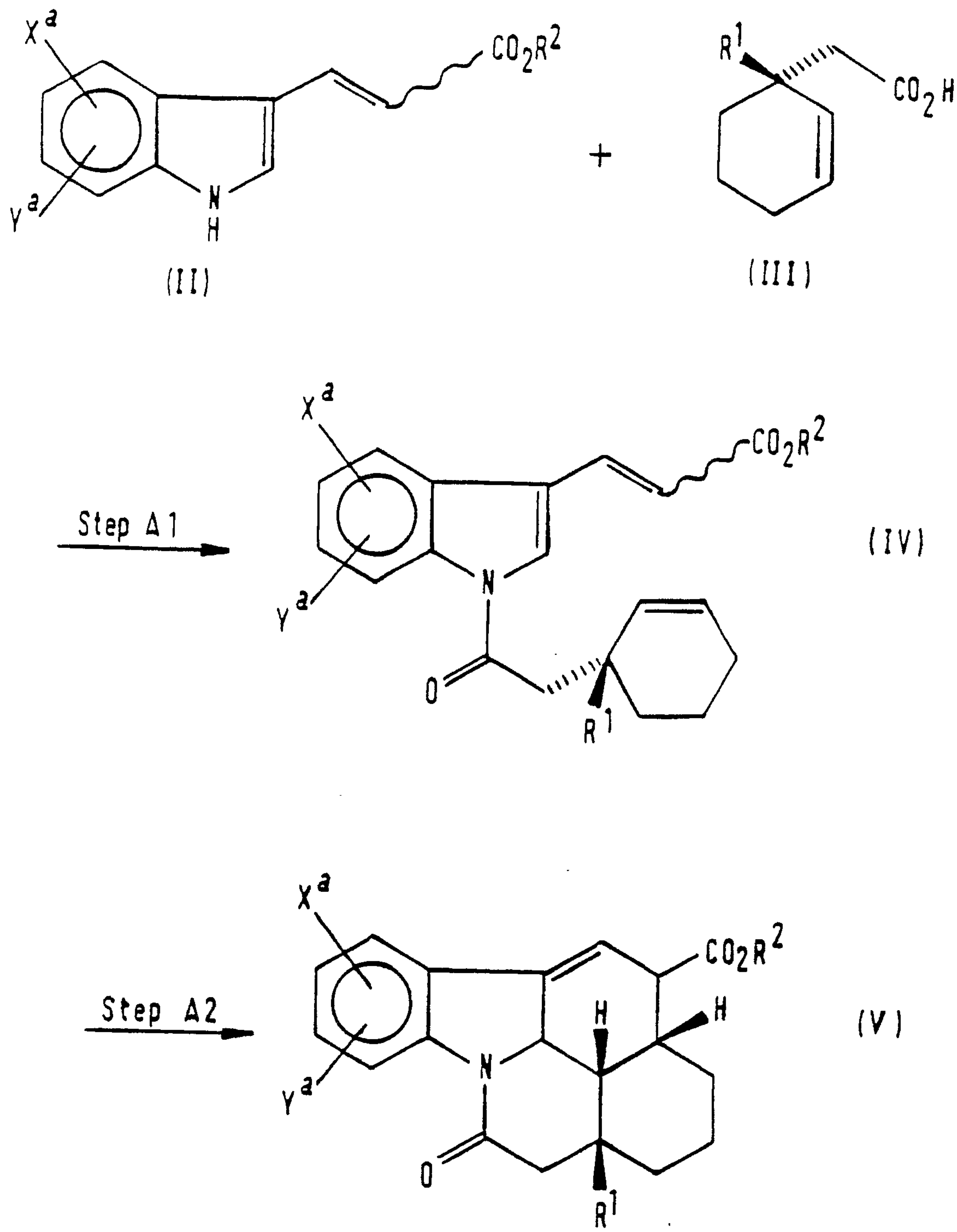


(II) + (III)

(IV)

(V)

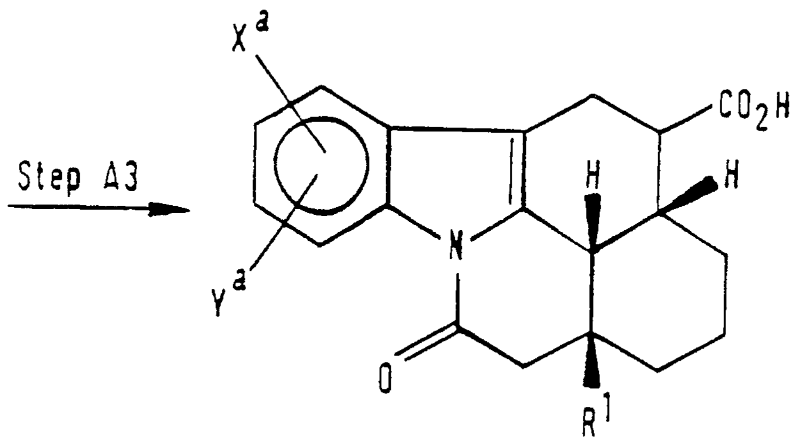


(VI)

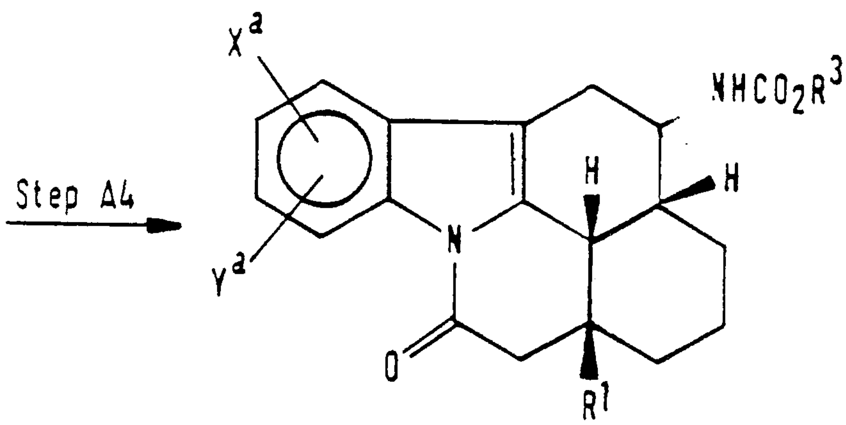


(VII)

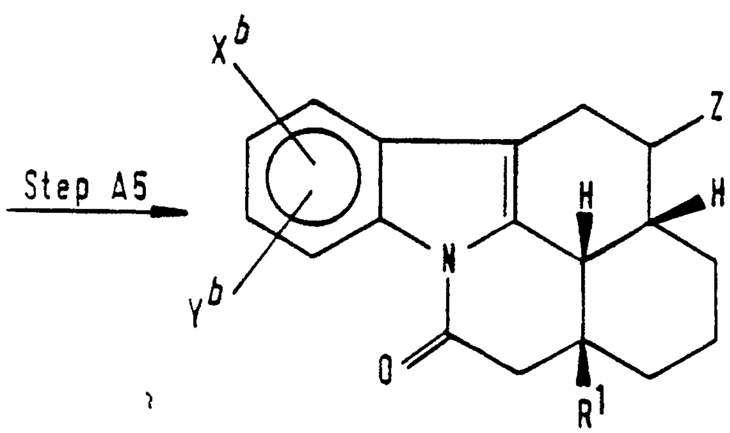


(I)

In the above formulae,
$R^1$, $R^2$, $R^3$, $X^a$, $Y^a$, $X^b$, $Y^b$ and Z are as defined above;

There is no particular restriction on the nature of the carboxy-protecting group which may be represented by $R^2$, and any carboxy-protecting group commonly used in organic chemistry for this type of reaction may equally be used here. Examples include alkyl groups containing from 1 to 4 carbon atoms, alkenyl groups containing from 3 to 6 carbon atoms and aralkyl groups containing from 7 to 10 carbon atoms; of these, we prefer alkyl groups containing from 1 to 4 carbon atoms and aralkyl groups containing from 7 to 10 carbon atoms.

Where $R^2$ or $R^3$ represents an alkenyl group containing from 3 to 6 carbon atoms, this may be a

straight or branched chain group and examples of such alkenyl groups include the allyl, methallyl, 2-buten-1-yl, 2-penten-1-yl and 2-hexen-1-yl groups. Of these, we prefer the alkenyl groups containing 3 or 4 carbon atoms.

Where $R^2$ or $R^3$ represents an aralkyl group containing from 7 to 10 carbon atoms, this is preferably an alkyl group having from 1 to 4 carbon atoms, which is substituted by a phenyl group. The phenyl group itself may optionally be substituted, for example with an alkyl group having from 1 to 4 carbon atoms, an alkoxy group having from 1 to 4 carbon atoms, a halogen atom (e.g. a fluorine, chlorine, bromine or iodine atom) or a nitro group. Examples of such aralkyl groups include the benzyl, phenethyl, 2-phenylpropyl, 3-phenylpropyl, 4-phenylbutyl, p -nitrobenzyl, p -methoxybenzyl, p -chlorobenzyl and p -methylbenzyl groups, of which we prefer the benzyl group which is unsubstituted or is substituted with at least one substituent selected from alkoxy groups having from 1 to 4 carbon atoms and nitro groups.

There is no particular restriction on the nature of the hydroxy-protecting group which may be represented by $X^a$ or $Y^a$, and any hydroxy-protecting group commonly used in organic chemistry for this type of reaction may equally be used here. Examples include the alkyl groups having from 1 to 4 carbon atoms, e.g. as exemplified above, the alkenyl groups having from 3 to 6 carbon atoms, e.g. as exemplified above, and the aralkyl groups having from 7 to 1C carbon atoms, e.g. as exemplified above, of which we prefer the alkyl groups and the aralkyl groups.

The various steps of the above Reaction Scheme may be carried out as follows:

Step A1:

The first step of this Reaction Scheme comprises reacting an indole compound of formula (II) or a reactive derivative thereof with an optically active cyclohexenylacetic acid of formula (III) or with a reactive derivative thereof to give a compound of formula (IV).

Suitable reactive derivatives of the compound of formula (III) include the acid halides (for example the acid chloride or acid bromide), the acid anhydride and mixed acid anhydrides of the compound of formula (III) with another organic carboxylic acid (such as acetic acid, propionic acid, butyric acid, benzoic acid or p -methylbenzoic acid) or with a monoalkyl carbonate in which the alkyl group has from 1 to 4 carbon atoms (such as the monomethyl carbonate, monoethyl carbonate and monoisobutyl carbonate). Of these, we prefer the acid halides. The reactive derivative can easily be prepared by reacting the compound of formula (III) with the corresponding halide [e.g. thionyl chloride, thionyl bromide, the acid chloride of the compound of formula (III), acetyl chloride, benzoyl chloride, methylformyl chloride, isobutylformyl chloride or the like].

The compound of formula (II) may be used as such or it may be employed in the form of an alkali metal salt, such as the sodium or potassium salt. Where the compound of formula (II) itself is used, the reaction is preferably carried out in the presence of an organic amine, such as triethylamine, pyridine or diethylaniline.

The reaction is normally and preferably effected in the presence of a solvent. There is no particular restriction on the nature of the solvent to be employed, provided that is has no adverse effect on the reaction or on the reagents involved. Examples of suitable solvents include: aromatic hydrocarbons, such as benzene, toluene and xylene; ethers, such as diethyl ether, tetrahydrofuran and dioxane; amides, especially fatty acid amides, such as dimethylformamide or dimethylacetamide; and ketones, such as acetone and methyl ethyl ketone. Of these, we prefer the ethers and the amides.

When the compound of formula (II) is employed in the presence of an organic amine, examples of preferred solvents also include halogenated hydrocarbons, especially halogenated aliphatic hydrocarbons, such as methylene chloride and chloroform.

The reaction can take place over a wide range of temperatures, and the precise reaction temperature is not critical to the invention. In general, we find it convenient to carry out the reaction at a temperature of from 0 °C to 50 °C (more preferably at about room temperature), although the preferred reaction temperature may vary depending on the nature of the starting materials. The time required for the reaction may also vary widely, depending on many factors, notably the reaction temperature and the nature of the reagents. However, provided that the reaction is effected under the preferred conditions outlined above, a period of from 30 minutes to 15 hours (more preferably from 1 hour to 10 hours) will usually suffice.

After completion of the reaction, the product may be recovered from the reaction mixture by conventional means, for example by adding water to the reaction mixture, extracting it with a water-immiscible organic solvent, and distilling off the solvent. If necessary, the product may be further purified by such conventional means as recrystallization or the various chromatography techniques, notably thin layer chromatography or column chromatography.

The starting material, the compound of formula (II), used in this step is either a known compound or can easily be prepared by well known methods [e.g. as desoribed in Heterocycles, 27 , 1253 (1988)].

The compound of formula (III) can be prepared by heating an optically active alcohol having the formula (VIII):

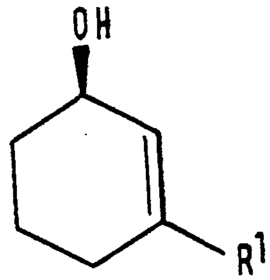

(VIII)

(in which $R^1$ is as defined above) at a temperature of from 50 ° C to 200 ° C for a period of from 10 hours to 100 hours in the presence of a lower alkyl orthoacetate (such as ethyl orthoacetate or methyl orthoacetate) and of an acid catalyst (e.g. a phenol such as 2-nitrophenol or 2,4-dinitrophenol; or a fatty acid such as propionic acid or butyric acid and by subsequent alkaline hydrolysis.

Other methods of preparing the compound of formula (IV) are also available, as is well known in the art, and so this step is optional in the process of the present invention.

Step A2:

In the second step of this Reaction Scheme, a compound of formula (V) is prepared by heating the compound of formula (IV) in the presence or absence of an inert solvent and in the presence or absence of a catalyst.

Examples of catalysts which may be employed in this step include Lewis acids, such as aluminium chloride, tin tetrachloride, boron trifluoride, ferric chloride and titanium tetrachloride.

Where a solvent is employed, there is no particular restriction on the nature of the solvent, provided that it has no adverse effect on the reaction or on the reagents involved. Examples of suitable solvents include: hydrocarbons, which may be aliphatic, aromatic or cycloaliphatic hydrocarbons, such as cyclohexane, benzene, toluene, xylene, mesitylene, tetrahydronaphthalene and biphenyl; ethers, such as diethyl ether, tetrahydrofuran, dioxane and biphenyl ether; amides, especially fatty acid amides, such as dimethylformamide and dimethylacetamide; and halogenated hydrocarbons, including halogenated aromatic and aliphatic hydrocarbons, such as methylene chloride, chloroform, chlorobenzene and the dichlorobenzenes. Of these, we prefer the hydrocarbons and halogenated hydrocarbons.

The reaction can take plaoe over a wide range of temperatures, and the precise reaction temperature is not critical to the invention. In general, although the preferred temperature will depend on the nature of the starting materials, we normally find it convenient to carry out the reaction at a temperature of from 50 ° C to 250 ° C (more preferably from 70 ° C to 200 ° C). The time required for the reaction may also vary widely, depending on many factors, notably the reaction temperature and the nature of the reagents. However, provided that the reaction is effected under the preferred conditions outlined above, a period of from 1 hour to 48 hours (more preferably from 10 hours to 30 hours) will usually suffice.

The reaction can be conducted either at ambient pressure or under superatmospheric pressure.

After completion of the reaction, the product may be recovered from the reaction mixture by conventional means, for example, by distilling off the solvent, or by adding water to the reaction mixture, extracting it with a water-immiscible organic solvent, and then distilling off the solvent. If necessary, the product may be further purified by such conventional techniques as recrystallization or the various chromatography techniques, notably preparative thin layer chromatography or column chromatography. In certain cases, the product can be used in the subsequent reaction without intermediate isolation.

This step enables the ^-ester compound of formula (Va) to be prepared stereoselectively from the compound of formula (IVa), in which the geometry of the double bond in the side chain is trans , and the a-ester compound of formula (Vb) to be prepared stereo-selectively from the compound of formula (IVb) in which the geometry of the double bond in the side chain is cis , as shown below:

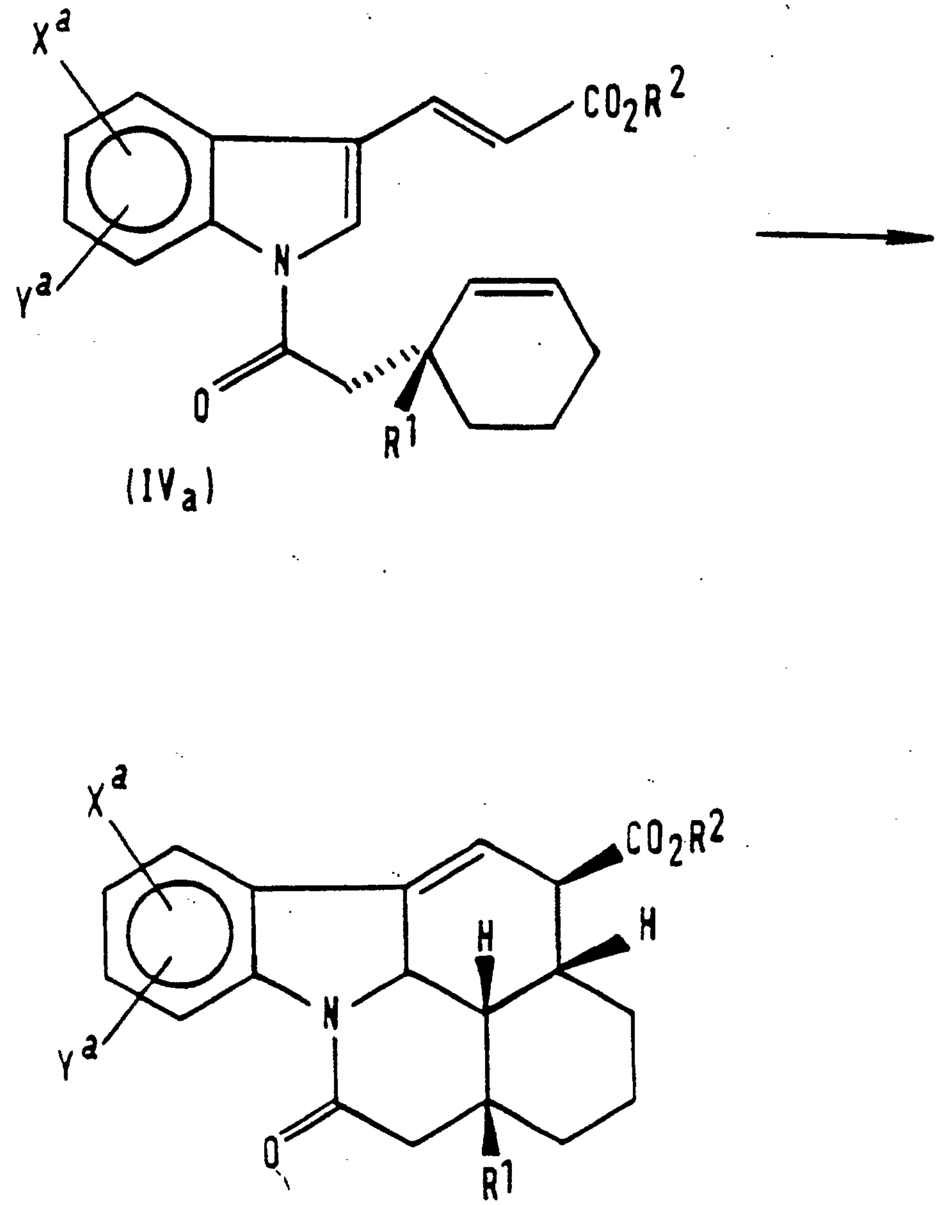

($IV_a$)

($V_a$)

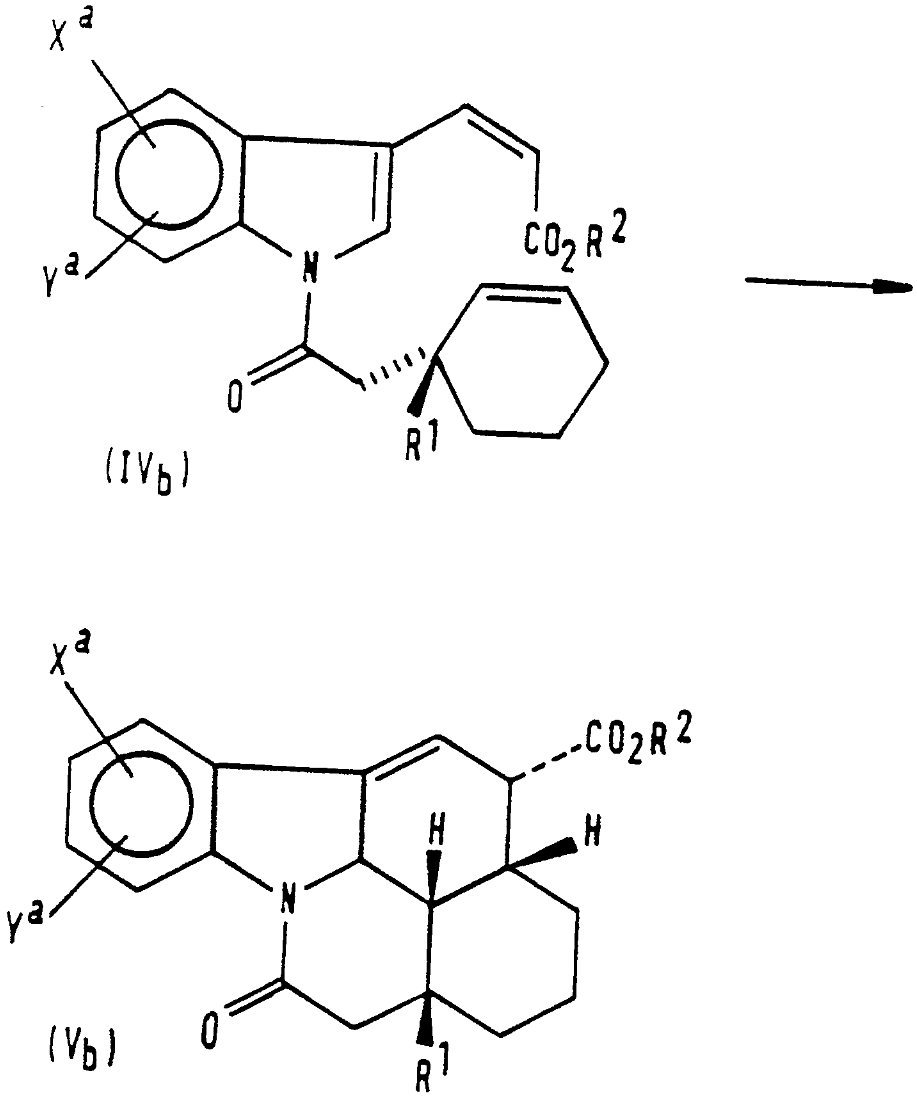

(IVb)

(Vb)

In some cases in this step it is possible to prepare the positional isomer of formula (V′) of the compound of formula (V), which the double bond is located at the same position as that of the compound (VI):

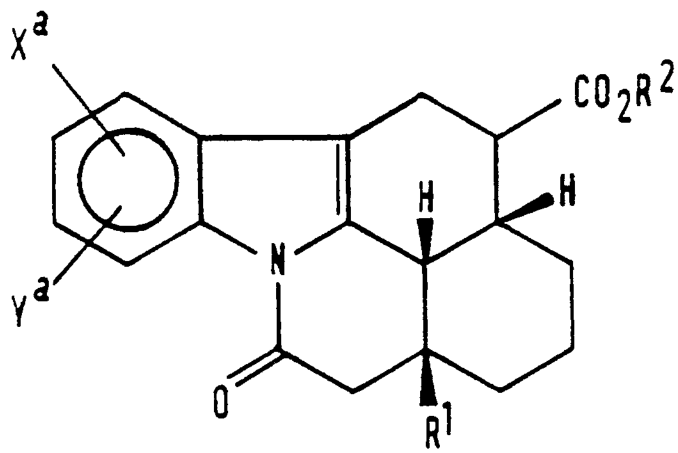

(V′)

Step A3:

In the third step of this Reaction Scheme, a compound of formula (VI) is prepared by isomerization, if necessary, of the compound. of formula (V) and then by removing the carboxy-protecting group $R^2$.

Isomerization can be effected by heating the compound of formula (V) in an inert solvent in the presence or absence of a catalyst (preferably in the presence of a catalyst) to produce the compound of formula (V′)

Examples of catalysts which may be employed include: metallic catalysts, such as palladium-on-charcoal, metallic silver, metallic palladium, tris(triphenylphosphine)rhodium chloride, rhodium chloride, cuprous chloride, ruthenium chloride and iron pentacarbonyl; inorganic acids, such as hydrochloric acid (or hydrogen chloride), sulphuric acid and phosphoric acid; organic acids, such as acetic acid, methanesulphonic acid and p -toluenesulphonic acid; and Lewis acids, such as aluminium chloride. Of these, we prefer the inorganic acids.

There is no particular restriction on the nature of the solvent to be employed, provided that it has no adverse effect on the reaction or on the reagents involved. Examples of suitable solvents include: aromatic hydrocarbons, such as benzene, toluene, xylene and mesitylene; ethers, such as diethyl ether, tetrahydrofuran and dioxane; amides, especially fatty acid amides, such as dimethylformamide and dimethylacetamide; and alcohols, such as methanol, ethanol and propanol. Of these, we prefer the ethers and the aromatic hydrocarbons.

Where the reaction conditions employed for isomerization are similar to those under which the protecting group $R^2$ may be removed, this may lead to removal of the protecting group $R^2$ in the course of carrying out the isomerization.

The reaction can take place over a wide range of temperatures, and the precise reaction temperature is not critical to the invention. In general, although the preferred reaction temperature will depend on such factors as the nature of the catalyst, we normally find it convenient to carry out the reaction at a temperature of from 40°C to 120°C (more preferably from 60°C to 100°C). The time required for the reaction may also vary widely, depending on many factors, notably the reaction temperature and the nature of the reagents. However, provided that the reaction is effected under the preferred conditions outlined above, a period of from 10 minutes to 10 hours (more preferably from 30 minutes to 5 hours) will usually suffice.

After completion of the reaction, the product can be recovered from the reaction mixture by conventional means, for example, by distilling off the solvent, or by adding water to the reaction mixture, extracting it with a water-immiscible organic solvent and distilling off the solvent. If necessary, the product can be further purified by such conventional means as recrystallization or the various chromatography techniques, notably preparative thin layer chromatography or column chromatography. However, the product can be used in the subsequent reaction without isolation.

Self-evidently, if the product of Step A2 is a compound of formula (V′), this isomerization step will be unnecessary.

Removal of the carboxy-protecting group represented by $R^2$ can usually be effected by hydrolysis. This hydrolysis can be conducted under the conditions conventionally used for hydrolysis of compounds of this type and there is no particular limitation. The reaction is preferably be carried out in the presence of an alkali, such as sodium hydroxide, potassium hydroxide, sodium carbonate or potassium carbonate (most preferably sodium hydroxide or potassium hydroxide) in an inert solvent such as an alcohol (e.g. methanol or ethanol) or an aqueous alcohol at a temperature of from room temperature to 80°C for a period of 30 minutes to 24 hours.

Where $R^2$ represents an aralkyl group, the compound of formula (VI) can also be prepared by catalytic reduction of the corresponding compound of formula (V) in the presence of hydrogen. The hydrogen pressure employed in the reaction may range from from ambient pressure to 5 times atmospheric pressure.

Examples of catalysts which may be employed include, for example, palladium-on-charcoal, platinum oxide and Raney nickel, preferably palladium-on-charcoal.

There is no particular restriction on the nature of the solvent to be employed, provided that it has no adverse effect on the reaction or on the reagents involved. Examples of suitable solvents include: alcohols, such as methanol and ethanol; ethers, such as diethyl ether, tetrahydrofuran and dioxane; and amides, especially fatty acid amides, such as dimethylformamide and dimethylacetamide. Of these, we prefer the ethers and alcohols.

The reaction can take place over a wide range of temperatures, and the precise reaction temperature is not critical to the invention. In general, we find it convenient to carry out the reaction at a temperature of from 0°C to 50°C (more preferably at about room temperature). The time required for the reaction may

also vary widely, depending on many factors, notably the reaction temperature and the nature of the reagents. However, provided that the reaction is effected under the preferred conditions outlined above, a period of from 1 hour to 15 hours will usually suffice.

Where $R^2$ represents a p -methoxybenzyl group, the protecting group can be removed in an inert solvent by treatment with an acid (e.g. a mineral acid such as hydrochloric acid, hydrobromic acid or sulphuric acid, or an organic carboxylic acid such as formic acid, acetic acid or trifluoroacetic acid) at a temperature of from room temperature to 100 °C for a period of from 30 minutes to 10 hours.

Where $R^2$ represents a $C_3$ - $C_6$ alkenyl group, the compound of formula (VI) can be prepared by reacting the corresponding compound of formula (V) with a proton donor compound in an inert solvent in the presence of a palladium complex. Examples of palladium complexes employed include complexes of 0 valent palladium, for example, complexes prepared by coordination of organic phosphorus compounds, such as triphenylphosphine, tributylphosphine and triethyl phosphite; preferably tetrakis(triphenylphosphine)-palladium(0).

Examples of proton donor compounds which may be employed include, for example: organic carboxylic acids, such as formic acids acetic acid and benzoic acid; phenols, such as phenol and cresol; and active methylene compounds, such as diethyl malonate and ethyl acetoacetate. Of these, we prefer the organic carboxylic acids.

There is no particular restriction on the nature of the solvent to be employed, provided that it has no adverse effect on the reaction or on the reagents involved. Examples of suitable solvents include: hydrocarbons, which may be aliphatic or aromatic hydrocarbons, such as hexane, and benzene; halogenated hydrocarbons, especially halogenated aliphatic hydrocarbons, such as methylene chloride and chloroform; ethers, such as diethyl ether, tetrahydrofuran and dioxane; alcohols, such as methanol, ethanol and t-butanol; ketones, such as acetone and methyl ethyl ketone; esters, such as methyl acetate and ethyl acetate; amides, especially fatty acid amides, such as dimethylformamide and dimethylacetamide; and sulphoxides, such as dimethyl sulphoxide. Of these, we prefer the halogenated hydrocarbons. A single one of these solvents may be used; alternatively, a mixture of any two or more may be used.

The reaction can take place over a wide range of temperatures, and the precise reaction temperature is not critical to the invention. In general, we find it convenient to carry out the reaction at a temperature of from 0 °C to 40 °C. The time required for the reaction may also vary widely, depending on many factors, notably the reaction temperature and the nature of the reagents. However, provided that the reaction is effected under the preferred conditions outlined above, a period of from 30 minutes to 24 hours will usually suffice. The reaction is preferably effected in an atmosphere of nitrogen with stirring or by allowing the mixture to stand.

After completion of the reaction, the product can be recovered from the reaction mixture by conventional means, for example, by cooling the reaction mixture and collecting the precipitated crystals by filtration, or where insoluble materials exist, by filtering, adding water, extracting with a water-immiscible organic solvent, acidifying the aqueous layer, extracting with a water-immiscible organic solvent and distilling off the solvent. If necessary, the product can be further purified by such conventional techniques as recrystallization or the various chromatography techniques, notably preparative thin layer chromatography and column chromatography.

Step A4:

In the fourth step of the Reaction Scheme, the compound of formula (VII) is prepared by reacting a compound prepared by heating an azide derivative of the compound of formula (VI) with a compound of formula (IX):

$R^3OH$ (IX)

wherein $R^3$ is as defined above (namely by carrying out a Curtius rearrangement).

The azidation reaction can be carried out by reacting a reactive derivative of the compound of formula (VI) with an alkali metal azide, suoh as lithium azide, sodium azide or potassium azide, in an inert solvent.

Examples of reactive derivatives of the compound of formula (VI) include, for example: acid halides, such as acid chloride or acid bromide of the corresponding compound; mixed acid anhydrides of the corresponding acid with an organic carboxylic acid, such as acetic acid, propionic acid or benzoic acid; and mixed acid anhydrides of the corresponding acid with a lower (i.e. $C_1$ - $C_4$) alkyl carbonate, such as monomethyl carbonate, monoethyl carbonate or monoisobutyl carbonate. Of these, we prefer the acid halides and mixed acid anhydrides with a monoalkyl carbonate.

There is no particular restriction on the nature of the solvent to be employed, provided that it has no

adverse effect on the reaction or on the reagents involved. Examples of suitable solvents include: aromatic hydrocarbons, such as benzene, toluene and xylene; ethers, such as diethyl ether, tetrahydrofuran and dioxane; amides, especially fatty acid amides, such as dimethylformamide and dimethylacetamide; ketones, such as acetone and methyl ethyl ketone; esters, such as methyl acetate and ethyl acetate; halogenated hydrocarbons, especially halogenated aliphatic hydrocarbons, such as methylene chloride and chloroform; and mixtures of water with an organic solvent. Of these, we prefer the ketones, aqueous ketones and ethers.

The reaction can take place over a wide range of temperatures, and the precise reaction temperature is not critical to the invention. In general, although the preferred reaction temperature will depend on the nature of the starting materials and other factors, we normally find it convenient to carry out the reaction at a temperature of from -10°C to 50°C (more preferably from 0°C to room temperature) The time required for the reaction may also vary widely, depending on many factors, notably the reaction temperature and the nature of the reagents. However, provided that the reaction is effected under the preferred conditions outlined above, a period of from 10 minutes to 10 hours (more preferably from 30 minutes to 3 hours) will usually suffice.

The desired compound can also be prepared by reacting the compound of formula (VI) with a phosphoric acid azide, such as diphenylphosphoric acid azide.

After completion of the reaction, the desired compound can be recovered from the reaction mixture by conventional means, for example, by adding water, extracting with an organic solvent and distilling off the solvent. If necessary, the produot may be further purified by such conventional techniques as recrystallization or the various chromatography techniques, notably column chromatography.

The reaction required to prepare the compound of formula (VII) from the azide compound prepared as described above can be carried out by a Curtius rearrangement by heating the azide compound in an inert solvent followed by reacting the product with the compound of formula (IX).

There is no particular restriction on the nature of the solvent to be employed, provided that it has no adverse effect on the reaction or on the reagents involved. Examples of suitable solvents include: aromatic hydrocarbons, such as benzene, toluene, xylene and mesitylene; ethers, such as diethyl ether, tetrahydrofuran and dioxane; amides, especially fatty acid amides, such as dimethylformamide and dimethylacetamide; halogenated hydrocarbons, especially halogenated aliphatic hydrocarbons, such as chloroform and 1,2-dichloroethane; and ketones, such as acetone and methyl ethyl ketone. Of these, we prefer the aromatic hydrocarbons.

Although the reaction temperature required for the Curtius rearrangement and for the reaction with the compound of formula (IX) will vary depending upon the nature of the starting materials employed, in general, we prefer that the reaction should be carried out at a temperature of from 50°C to 200°C (more preferably from 70°C to 150°C). The time required for the reaction may likewise vary widely depending upon the reaction temperature and other factors, but, provided that the reaction is carried out under the preferred conditions described above, a period of from 15 minutes to 6 hours (more preferably from 30 minutes to 3 hours) will normally suffice for the Curtius rearrangement and from 30 minutes to 15 hours (more preferably from 2 hours to 10 hours) will normally suffice for the reaction with the compound of formula (IX).

After completion of the reaction, the desired compound may be recovered from the reaction mixture by conventional means, for example, by distilling off the solvent or by adding water, extracting with a water-immiscible organic solvent and distilling off the solvent. If necessary, the product can be further purified by such conventional techniques as recrystallization or the various chromatography techniques, notably preparative thin layer chromatography or column chromatography.

In particular, the desired compound can be obtained from the mother liquor in a form having a high optical purity by recrystallization from a mixture of methylene chloride and ethyl acetate.

Step A5:

In the fifth step of this Reaction Scheme, a compound of formula (I) is prepared by: removing the hydroxy-protecting group, if any, in the groups represented by $X^a$ and $Y^a$; removing the group of formula $-CO_2R^3$ from the amino group at the 4-position; and converting the amino group to an alkylamino or cyclic amino group. These reactions may be carried out in any suitable order.

Where the hydroxy-protecting group is an alkyl group, it may be removed by reacting the corresponding compound of formula (VII) with an acid in an inert solvent.

Suitable acids for use in this reaction include, for example: Lewis acids, such as boron tribromide, boron trichloride and aluminium chloride; and mineral acids, such as hydrobromic acid, hydroiodic acid and

sulphuric acid. Of these, we prefer the Lewis acids, such as boron tribromide.

There is no particular restriction on the nature of the solvent to be employed, provided that it has no adverse effect on the reaction or on the reagents involved. Examples of suitable solvents include: hydrocarbons, such as benzene, toluene and xylene; halogenated hydrocarbons, espeoially halogenated aliphatic hydrocarbons, such as methylene chloride, chloroform and 1,2-dichloroethane; and ethers, such as diethyl ether, tetrahydrofuran and dioxane. Of these, we prefer the halogenated hydrocarbons.

The reaction can take place over a wide range of temperatures, and the precise reaction temperature is not critical to the invention. In general, we find it convenient to carry out the reaction at a temperature of from 0°C to 80°C (more preferably, 0°C to 50°C). The time required for the reaction may also vary widely, depending on many factors, notably the reaction temperature and the nature of the reagents. However, provided that the reaction is effected under the preferred conditions outlined above, a period of from 1 hour to 24 hours (more preferably from 3 hours to 20 hours) will usually suffice.

The desired product can be recovered from the reaction mixture by distilling off the solvent or by extracting the reaction mixture with a water-immiscible solvent, washing it with water, drying it over anhydrous magnesium sulphate and distilling off the solvent. If necessary, the product may be further purified by such conventional means as recrystallization or the various chromatography techniques, notably column chromatography.

Where the hydroxy-protecting group is an aralkyl group, the protecting group may be removed by catalytic reduction of the corresponding compound of formula (VII). This reaction can be carried out in a similar manner to that of the deprotection reaction in the third step wherein $R^2$ represents an aralkyl group.

Where the hydroxy-protecting group is an alkenyl group, the protecting group may be removed by reacting the corresponding compound of formula (VII) with a proton donating compound in the presence of a palladium complex. This reaction can be carried out in a similar manner to that of the deprotection reaction in the third step wherein $R^2$ represents an alkenyl group.

The removal of the group of formula $-CO_2R^3$ may be carried out in a similar manner to that described above for the deprotection of $R^2$. Where the reaction conditions for the removal of the hydroxy-protecting group are similar to those for the removal of the group of formula $-CO_2R^3$, the hydroxy-protecting group and the group of formula $-CO_2R^3$ may be removed simultaneously.

The conversion of the amino group to an alkylamino or cyclic amino group can be carried out by reacting the corresponding compound of formula (I) in which Z represents said group of formula $-NR^aR^b$ and $R^a$ and $R^b$ both represent hydrogen atoms with a compound of formula (X) or (XI):

$R^4$-Q (X)

or

Q-A-Q′ (XI)

(wherein $R^4$, A, Q and Q′ are as defined above).

If necessary, prior to this reaction, any hydroxy group represented by $X^b$ and/or $Y^b$ may be protected.

Examples of the halogen atom which may be represented by Q and Q′ include the chlorine, bromine and iodine atoms.

The nature of the compound of formula (X) and (XI) will, of course, depend on the nature of the group Z which it is desired to form. However, examples of compounds of formula (X) or (XI) include ethyl bromide, propyl bromide, butyl bromide, methyl iodide, ethyl iodide, butyl iodide, 2-chloroethanol, 2-iodoethanol, 3-iodopropanol, 4-iodobutanol, 1,2-diiodoethane, 1,3-diiodopropane, 1,4-diiodobutane, 1,4-dichlorobutane, 1,5-diiodopentane, 1,5-dichoropentane, 2-chloroethyl ether, 2-chloroethylthio ether, 2-iodoethyl ether, 2-iodoethylthio ether and N-benzyloxycarbonyl-bis(2-chloroethyl)amine.

The reaction employed for protection of the hydroxy groups represented by $X^b$ and/or $Y^b$ may be any conventional hydroxy-protecting reaction, for example by reacting the hydroxy compound with a halide, such as methyl iodide, allyl chloride, allyl bromide, benzyl chloride or benzyl bromide, in the presence of a base, for example sodium hydride.

The reaction is preferably carried out in the presence of an base. The nature of the base employed in the reaction is not particularly critical, and any base of the type commonly used in this sort of reaction may equally be employed here, provided that it has no adverse effect on the reaction or on the reagents. Examples of such bases include: alkali metal carbonates and hydrogencarbonates, suoh as sodium hydrogencarbonate, potassium hydrogencarbonate, sodium carbonate and potassium carbonate; and organic amines, such as triethylamine, pyridine and diethylaniline. Of these, we prefer the alkali metal carbonates and hydrogencarbonates.

There is no particular restriotion on the nature of the solvent to be employed, provided that it has no adverse effect on the reaction or on the reagents involved. Examples of suitable solvents include: aromatic hydrocarbons, such as benzene, toluene and xylene; ethers, such as diethyl ether, tetrahydrofuran and

dioxane; amides, especially fatty acid amides, such as dimethylformamide and dimethylacetamide; alcohols, such as methanol, ethanol and propanol; halogenated hydrocarbons, especially halogenated aliphatic hydrocarbons, such as methylene chloride, chloroform and 1,2-dichloroethane; and esters, such as methyl acetate and ethyl acetate. Of these, we prefer the aromatic hydrocarbons.

The reaction can take place over a wide range of temperatures, and the precise reaction temperature is not critical to the invention. In general, although the preferred temperature will depend on various factors, suoh as the nature of the starting materials, we normally find it convenient to carry out the reaction at a temperature of from room temperature to 200°C (more preferably from 50°C to 150°C). The time required for the reaction may also vary widely, depending on many factors, notably the reaction temperature and the nature of the reagents. However, provided that the reaction is effected under the preferred conditions outlined above, a period of from 30 minutes to 48 hours (more preferably from 3 hours to 20 hours) will usually suffice.

Where Z represents a dimethylamino group, the desired product may be prepared by such conventional means as treating the corresponding amino compound with formalin-formic acid or formalin-sodium borohydride.

Where Z represents a piperazinyl group, an amino-protecting group, such as a benzyloxycarbonyl group, can be removed by catalytic hydrogenation using, for example, palladium-on-carbon as the catalyst.

After completion of the reaction, the desired compound can be recovered from the reaction mixture by conventional means, for example, by distilling off the solvent, or by adding water, extracting with a water-immiscible organic solvent and distilling off the solvent. If necessary, it can be further purified by such conventional means as recrystallization or the various chromatography techniques, notably thin layer chromatography or column chromatography.

Alternatively, a compound of formula (VIII) wherein $R^1$ represents a hydrogen atom, i.e. a compound of formula (VIII ), can be prepared by using the method illustrated in the following Reaction Scheme B:

Reaction Scheme: B

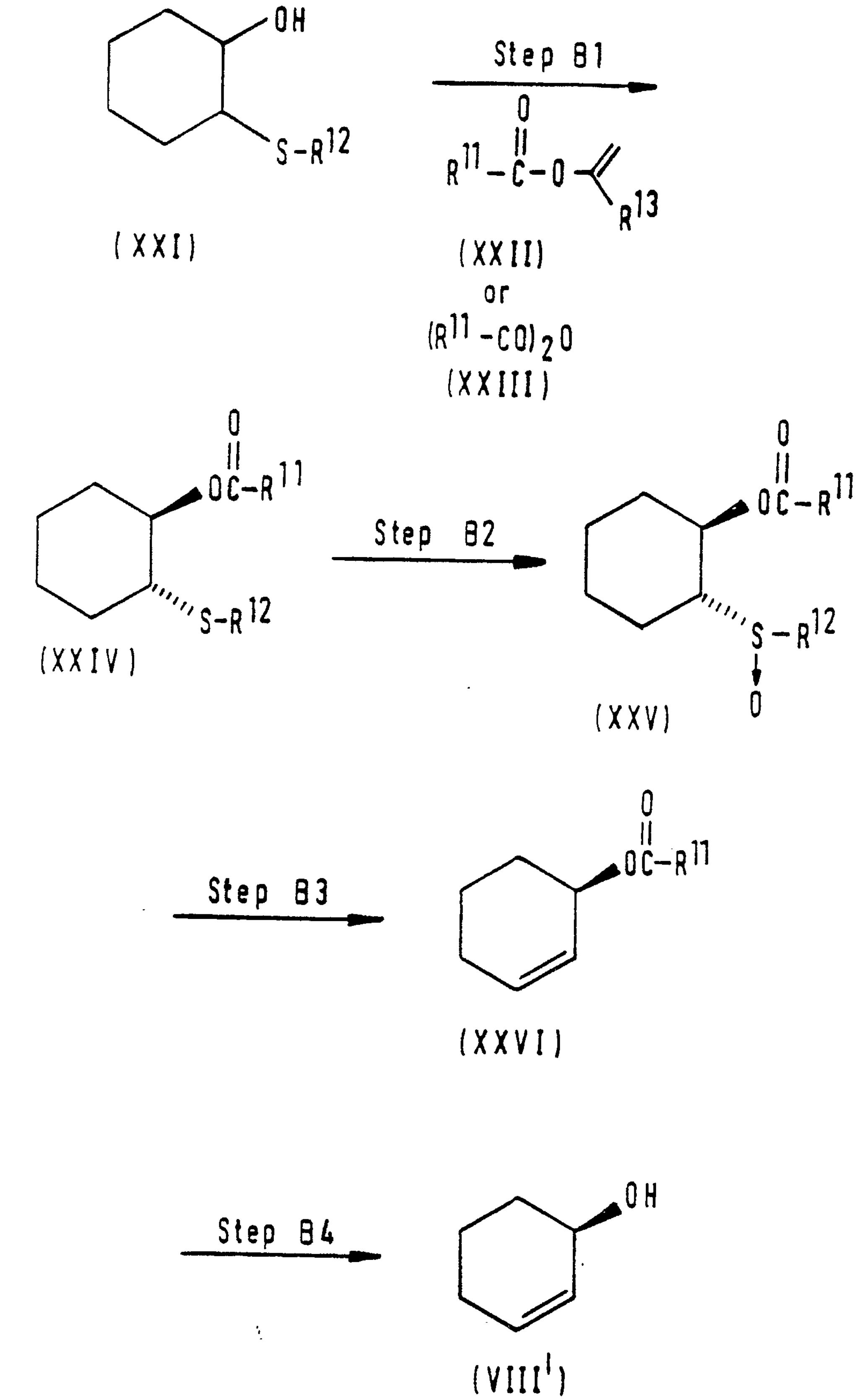


(XXI)

(XXIV)

(XXV)

(XXVI)

(VIII')

In the above formulae:

$R^{11}$ represents an alkyl group having from 1 to 4 carbon atoms, an unsubstituted phenyl group or a substituted phenyl group having at least one substituent selected from alkyl groups having from 1 to 4 carbon atoms, alkoxy groups having from 1 to 4 carbon atoms and halogen atoms;

$R^{12}$ represents an alkyl group having from 1 to 4 carbon atoms, an unsubstituted phenyl group or a substituted phenyl group having at least one substituent selected from alkyl groups having from 1 to 4 carbon atoms, alkoxy groups having from 1 to 4 carbon atoms, halogen atoms and nitro groups; and

$R^{13}$ represents a hydrogen atom or an alkyl group having from 1 to 4 carbon atoms.

Step B1:

In this step of the Reaction Scheme, a dl -trans -cyclohexanol compound of formula (XXI) is reacted with a vinyl carboxylate compound of formula (XXII) or with an carboxylic anhydride compound of formula (XXIII) in the presence of a lipase to give a compound of formula (XXIV). Examples of the lipase which may be employed in this step include Pseudomonas Lipase, Porcine Pancreatic Lipase, Yeast Lipase, Aspergillus Lipase, Candida Lipase, Rhizopus Lipase or Mucor Lipase, preferably Pseudomonas Lipase.

Preferred examples of the compound of formula (XXII) which may be employed in this step include isopropenyl acetate, vinyl acetate, isopropenyl propionate, vinyl propionate and isopropenyl benzoate. Preferred examples of the compound of formula (XXIII) which may be employed in this step include acetic anhydride, propionic anhydride and benzoic anhydride.

The reaction is normally and preferably effected in the presence of an organic solvent. There is no particular restriction on the nature of the solvent to be employed, provided that it has no adverse effect on the reaction or on the reagents involved. Examples of suitable solvents include: aliphatic hydrocarbons, such as pentane and hexane; cyclic hydrocarbons, such as cyclohexane; aromatic hydrocarbons, such as benzene, toluene and xylene; ethers, such as diethyl ether, diisopropyl ether, tetrahydrofuran and dioxane; amides, especially fatty acid amides, such as dimethylformamide and dimethylacetamide; ketones, such as acetone and methyl ethyl ketone; nitriles, such as acetonitrile; and halogenated hydrocarbons, such as methylene chloride, dichloroethane, chloroform and carbon tetrachloride. Of these, we prefer the ethers, aliphatic hydrocarbons, aromatic hydrocarbons, ketones and nitriles and particularly prefer the ethers, aliphatic hydrocarbons, ketones and nitriles

The reaction can take plaoe over a wide range of temperatures, and the precise reaction temperature is not critical to the invention. In general, we find it convenient to carry out the reaction at a temperature of from 0°C to 50°C (more preferably at about room temperature), although the preferred reaction temperature may vary depending on the nature of the starting materials. The time required for the reaction may also vary widely, depending on many factors, notably the reaction temperature and the nature of the reagents. However, provided that the reaction is effected under the preferred conditions outlined above, a period of from 1 hour to 48 hours (more preferably from 5 hour to 30 hours) will usually suffice.

After completion of the reaction, the product may be recovered from the reaction mixture by conventional means, for example by filtering off the lipase and distilling off the solvent, or by adding water to the reaction mixture, extracting it with a water-immiscible organic solvent, distilling off the solvent and purifying the residue by such conventional means as the various chromatography techniques, notably thin layer chromatography or column chromatography.

This step is very useful to obtain an optically active compound of formula (XXIV) because the yield and selectivity of the reaction are excellent and separation of an optically active compound of formula (XXIV) from unreacted reagent of formula (XXI′) are very easy.

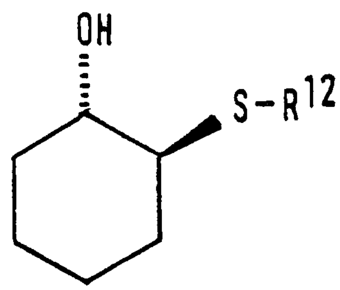

(XXI')

Step B2:

In this step of the Reaction Scheme, a compound of formula (XXV) is prepared by oxidizing the compound of formula (XXIV).

Examples of oxidizing agents which may be employed in this step include alkali metal perhalogenates, such as sodium perchlorate, sodium periodate and potassium periodate; hydrogen peroxide; and percarboxylic acids and salts thereof, such as peracetic acid, m -chloroper benzoic acid and magnesium monoperoxyphthalate. Of these, the alkali metal perhalogenates are most preferred.

The reaction is normally and preferably effected in the presence of a solvent. There is no particular restriction on the nature of the solvent to be employed, provided that it has no adverse effect on the reaction or on the reagents involved. Where an alkali metal perhalogenate is used, examples of suitable solvents include: alcohols, such as methanol and ethanol; water; and aqueous alcohols. Where hydrogen peroxide or a percarboxylic acid or salt thereof is used, examples of suitable solvents include halogenated hydrocarbons, especially halogenated aliphatic hydrocarbons, such as methylene chloride, chloroform and carbon tetrachloride.

The reaction can take place over a wide range of temperatures, and the precise reaction temperature is not critical to the invention. In general, although the preferred temperature will depend on the nature of the starting materials, we normally find it convenient to carry out the reaction at a temperature of from 0 °C to 50 °C (more preferably at about room temperature).

The time required for the reaction may also vary widely, depending on many factors, notably the reaction temperature and the nature of the reagents. However, provided that the reaction is effected under the preferred conditions outlined above, a period of from 30 minutes to 48 hours (more preferably from 1 hour to 24 hours) will usually suffice.

After completion of the reaction, the product may be recovered from the reaction mixture by conventional means, for example, by evaporating the solvent, if necessary adding water to the residue, extracting it with a water-immiscible organic solvent, and then distilling off the solvent. If necessary, the product may be further purified by such conventional techniques as recrystallization or the various chromatography techniques, notably preparative thin layer chromatography or column chromatography. In certain cases, the product can be used in the subsequent reaction without intermediate isolation.

Step B3:

In this step of the Reaction Scheme, a compound of formula (XXVI) is prepared by heating the compound of formula (XXV) under atmospheric pressure or under a reduced pressure (for example from about 10 mmHg - 100 mmHg, 1333 Pa to 13330 Pa) in the presence or absence of an inert solvent and in the presence or absence of a sulphenic acid trapping agent to produce the compound of formula (XXVI)

There is no particular restriction on the nature of the solvent which may be employed, provided that it has no adverse effect on the reaction or on the reagents involved. Examples of suitable solvents include: aromatic hydrocarbons, such as benzene, toluene, xylene and mesitylene; amides, especially fatty acid amides, such as dimethylformamide and dimethylacetamide; and halogenated hydrocarbons, such as chloroform and carbon tetrachloride. Of these, we prefer the aromatic hydrocarbons and halogenated

hydrocarbons.

Examples of the sulphenic acid trapping agent which may be employed in this step include alkaline earth metal carbonates, such as calcium carbonate and barium carbonate, and cycloalkenes, such as cyclohexene.

The reaction can take place over a wide range of temperatures; however, we normally find it convenient to carry out the reaction at a temperature of from 50 °C to 250 °C (more preferably from 100 °C to 200 °C). The time required for the reaction may also vary widely, depending on many factors, notably the reaction temperature and the nature of the reagents. However, provided that the reaction is effected under the preferred conditions outlined above, a period of from 1 hour to 24 hours (more preferably from 2 hours to 10 hours) will usually suffice.

Step B4:

In this step of the Reaction Scheme, a compound of formula (VIII′) is prepared by hydrolyzing the compound of formula (XXVI) in the presence of a base.

There is no particular restriction on the nature of the base which may be employed in this step, provided that it has no adverse effect on any other part of the molecule, and any base conventionally employed in this type of reaction may equally be employed here. Examples of suitable bases include; alkali metal hydroxides, such as sodium hydroxide and potassium hydroxide; alkali metal carbonates, such as sodium carbonate and potassium carbonate; and alkali metal alkoxides, such as sodium methoxide, sodium ethoxide and potassium t-butoxide. Of these, the alkali metal hydroxides are preferred.

The reaction is normally and preferably effected in the presence of a solvent. There is no particular restriction on the nature of the solvent to be employed, provided that it has no adverse effect on the reaction or on the reagents involved. Examples of suitable solvents include: alcohols, such as methanol and ethanol; water; and aqueous alcohols.

The reaction can take place over a wide range of temperatures, and the precise reaction temperature is not critical to the invention. In general, although the preferred temperature will depend on the nature of the starting materials, we normally find it convenient to carry out the reaction at a temperature of from 0 °C to 80 °C (more preferably at about room temperature). The time required for the reaction may also vary widely, depending on many factors, notably the reaction temperature and the nature of the reagents. However, provided that the reaction is effected under the preferred conditions outlined above, a period of from 1 hour to 48 hours (more preferably from 3 hours to 24 hours) will usually suffice.

After completion of the reaction, the product may be recovered from the reaction mixture by conventional means, for example, by evaporating the off solvent, if necessary adding water to the resulting residue, extracting it with a water-immiscible organic solvent, and then distilling off the solvent. If necessary, the product may be further purified by such conventional techniques as the various chromatography techniques, notably preparative thin layer chromatography or column chromatography.

When carried out in accordance with the preferred conditions outlined above, the process of the present invention enables one to produce optically active compounds of formula (I), whioh have an excellent anti-arrhythmic activity, in notably high yields and of high purity by using the optically active compound of formula (III) as the starting material and carrying out simple procedures.

## BIOLOGICAL ACTIVITY

The compounds of the present invention have shown an anti-arrhythmic activity which is substantially and significantly greater than that of the prior art racemic compounds. This is demonstrated by the following test, which compares the effects of ( 3a R ,4 R , 12a R , 12b S ) 4-amino-2,3,3a,4,5,12,12a,12b-octahydro-6-hydroxyindolo[3,2,1,-ij]benz[de]quinolin-11 (1 H )-one hydrochloride sesquihydrate (the compound prepared as described in Example 1) with those of the racemate of this compound on arrhythmias caused by myocardial ischemia in dogs.

Male Beagles weighing 8 - 12 kg were anesthetized with sodium pentobarbital (30 mg/kg i.v.). The animal was placed in a supine position, and the left carotid artery was isolated from the surrounding tissue. A metal cannula was then introduced from the left carotid artery and its tip was placed at the coronary orifice. A glass bead having a diameter of 1.7 mm was injected through the metal cannula into the left coronary artery. The glass bead injection produced a ST segment elevation, which was followed by

ventricular arrhythmias due to myocardial infarction.

The dog was used in a conscious state for 24 hours after myocardial ischemia developed, when the arrythmic ratio exceeded 70%. The arrhythmic ratio was calculated by dividing the number of arrhythmias by the total heart beats in one minute.

Figures 1 and 2 of the accompanying drawings show the course of the arrhythmias with time following a single intravenous administration of the compound of Example 1 and its racemate, respectively. Both agents decreased the number of arrhythmias per minute. However, the compounds of Example 1 at 2.5 mg/kg i.v. was almost comparable with its racemate at 5.0 mg/kg i.v. in suppressing arrhythmias These data suggest that the compound of Example 1 is about twice as potent as its racemate in its anti-arrhythmic action.

The compounds of the invention may be formulated, for therapeutic use, into various conventional formulations, the precise formulation chosen being dependent upon the route of administration. For example, for oral administration, the compounds can be formulated as tablets, capsules, powders or syrups. For parenteral administration, they can be formulated with injectible media for subcutaneous or intravenous injections. They can also be formulated as suppositories. The compounds will normally and preferably be mixed with various conventional carriers and diluents, for example: solubilizing agents, suspending agents, excipients, binders, disintegrating agents and optionally other therapeutically active compounds. The dosage will vary, depending upon the symptoms, age and body weight of the patient, as well as the nature and severity of the disorder and the route and form of administration, but a suitable dose for an adult human patient would be within the range from 20 to 200 mg per day, which can be administered as a single dose or in divided doses, e.g. 2 or 3 doses.

The invention is further illustrated by the following Examples, which illustrate the preparation of various of the compounds of the present invention. The preparation of certain of the starting materials used in these Examples is illustrated by the subsequent Preparations.

## EXAMPLE 1

(3aR,4R,12aR,12bS) 4-Amino-2,3,3a,4,5,12,12a,12b-octahydro-6-hydroxyindolo[3,2,1,-ij]benz[de]quinolin-11-(1H)-one hydrochloride sesquihydrate

1(a) (R) Benzyl (E)-3-[1-(2-cyclohexen-1-yl)acetyl-4-methoxy-1H-indol-3-yl]propenoate

2.18 g of sodium hydride (as a 55% by weight suspension in mineral oil) were added to a solution of 27.7 g of benzyl ( E )-3-(4-methoxy-1 H -indol-3-yl)-propenoate in 180 ml of tetrahydrofuran, whilst ice-cooling, and the mixture was stirred for 30 minutes. At the end of this time, a solution of 14.42 g of ( R )-2-cyclohexenylacetyl chloride (prepared as described in Preparation 2) in 7.7 ml of tetrahydrofuran was added to the mixture, and the mixture was stirred for 1 hour, whilst ice-cooling. After this, the reaction mixture was poured into 900 ml of dilute aqueous hydrochloric acid and then extracted several times with methylene chloride. The combined extracts were washed with water and with a saturated aqueous solution of sodium chloride, in that order, and dried over anhydrous sodium sulphate. The solvent was then removed by distillation under reduced pressure. The resulting residue was dissolved in 200 ml of methylene chloride, and then 400 ml of diisopropyl ether were added to the resulting solution. The precipitate which deposited was dried, to afford 34.13 g of the title compound, melting at 122 - 123 °C.
Mass spectrum (m/e): 429 ($M^+$).

| Elemental analysis: | |
|---|---|
| Calculated for $C_{27}H_{27}NO_4$: | |
| | C, 75.50%; H, 6.34%; N, 3.26%. |
| Found: | C, 75.43%; H, 6.28%; N, 3.27%. |

Specific rotation: $[a]_D^{25}$ = -55.6° (c = 1.01, $CHCP_3$).
Nuclear Magnetic Resonance Spectrum ($CDCP_3$) w ppm:

1.0 - 2.3 (6H, multiplet);
2.6 - 3.0 (3H, multiplet);
3.93 (3H, singlet);
5.25 (2H, singlet);
5.6 - 5.9 (2H, multiplet);
6. 50 (1H, doublet, J = 17 Hz);
6.77 (1H, doublet, J = 8 Hz);
7.1 - 7.6 (6H, multiplet);
7.70 (1H, singlet);
8.12 (1H, doublet, J = 8 Hz);
8.33 (1H, doublet, J = 17 Hz).

Infrared Absorption Spectrum (KBr) $n_{max}$ $cm^{-1}$:
1704.

1(b) (3aR,4R,12aR,12bS) 1,2,3,3a,4,5,11,12,12a,12b-Decahydro-6-methoxy-11-oxoindolo-3,2,1-ij]benz[de]-quinoline-4-carboxylic acid

32.2 g of ( R ) benzyl ( E )-3-[1-(2-cyclohexen-1-yl)acetyl-4-methoxy-1 H -indol-3-yl]propenoate [prepared as described in step (a) above] were suspended in 140 ml of mesitylene, and the suspension was heated under reflux for 24 hours. At the end of this time, it was allowed to cool to 60° C, and then 8.5 ml of a 4N solution of hydrogen chloride in dioxane were added. The mixture was then stirred at 85° C for 1 hour, after which the solvent was removed by distillation under reduced pressure. The resulting residue was purified by column chromatography through silica gel, and 36.7 g of an ester compound were obtained, as a yellow oil, from those fractions eluted with a 3 : 1 by volume mixture of cyolohexane and ethyl acetate. The whole of this oily product was dissolved in 320 ml of tetrahydrofuran, and the solution was stirred vigorously at room temperature for 8 hours in the presence of 1.70 g of 10% w/w palladium-on-charcoal and in an atmosphere of hydrogen. 250 ml of dimethylformamide were then added to the reaction mixture, and the mixture was stirred at 60° C for 1 hour and then filtered to remove the catalyst. The solvent was removed from the filtrate by distillation under reduced pressure, and the crystalline residue was washed with ethyl acetate. After the residue had been dried, 21.4 g of the title compound were obtained, melting at 288 - 292° C.

Specific rotation: $[a]_D^{24}$ = +104.9° (c = 1.02, dimethylformamide).
Mass spectrum (m/e): 339 ($M^+$).
Nuclear Magnetic Resonance Spectrum (hexadeuterated dimethyl sulphoxide) w ppm:
0.6 - 1.7 (6H, multiplet);
2.10 - 2.25 (1H, multiplet);
2.40 - 2.55 (2H, multiplet);
2.8 - 2.9 (2H, multiplet);
3.05 - 4.35 (3H, multiplet);
3.86 (3H, singlet);
6.78 (1H, doublet, J = 8 Hz);
7.16 (1H, triplet, 8 Hz);
7.83 (1H, doublet, J = 8 Hz);
Infrared Absorption Spectrum (KBr) $n_{max}$ $cm^{-1}$:
3100, 1736, 1671.

1(c) ( 3aR,4R,12aR,12bS) 4-Benzyloxycarbonylamino-2,3,3a,4,5,12,12a,12b-octahydro-6-methoxyindolo-[3,2,1-ij]benz[de]quinolin-11(1H)-one

5.4 ml of triethylamine and 4.6 ml of ethyl chloroformate were added to a suspension of 11.05 g of (3a R ,4 R ,12a R ,12b S ) 1,2,3,3a,4,5,11,12,12a,12b-decahydro-6-methoxy-11-oxoindolo[3,2,1-ij]benz[de]-quinolin-4-carboxylic acid [prepared as described in step (b) above] in 88 ml of acetone, whilst ice-cooling. The mixture was then stirred for 30 minutes, after which a solution of 3.14 g of sodium azide in 19 ml of water was added. The mixture was then stirred for 1 hour, whilst ice-cooling, after which it was poured into water and extracted with methylene chloride. The extracts were washed with water and with a saturated aqueous solution of sodium chloride, in that order, and dried over anhydrous sodium sulphate. The solvent

was then removed by distillation under reduced pressure, to afford 16.4 g of an acid azide as colourless crystals. The whole of this acid azide was suspended in 100 ml of toluene, and the suspension was heated under reflux for 1.5 hours. At the end of this time, 8.8 ml of benzyl alcohol were added to the suspension and the mixture was heated under reflux for 5 hours. The reaction mixture was then freed from the solvent by distillation under reduced pressure, and the residue was purified by column chromatography through silica gel using a 3 : 1 by volume mixture of cyclohexane and ethyl acetate as the eluent. The resulting crude product was reprecipitated from a mixture of methylene chloride and diisopropyl ether to afford 10.8 g of the title compound. The optical purity of the resulting product was about 70% ee. After recrystallization from a mixture of methylene chloride and ethyl acetate to remove 2.63 g of a racemate, 7.87 g of the title compound, melting at 222 - 224° C and having an optical purity of more than 96% ee were obtained from the mother liquor.
Specific rotation: $[a]_D^{24}$ = +88.6 (c = 1.00, $CHCP_3$).
Mass spectrum (m/e): 444 ($M^+$).

| Elemental analysis: | |
|---|---|
| Calculated for $C_{27}H_{28}N_2O_4$: | |
| | C, 72.95%; H, 6.35%; N, 6.30%. |
| Found: | C, 72.87%; H, 6.27%; N, 6.32%. |

Infrared Absorption Spectrum (KBr) $n_{max}$ $cm^{-1}$:
3370, 1721, 1711.
Nuclear Magnetic Resonance Spectrum ($CDCP_3$) w ppm:
0.9 - 1.7 (6H, multiplet);
2.1 - 2.4 (2H, multiplet);
2.67 (1H, doublet of doublets, J = 2 & 17 Hz);
2.9 - 3.2 (4H, multiplet);
3.87 (3H, singlet);
4.05 - 4.15 (1H, multiplet);
5.0 - 5.2 (3H, multiplet);
6.69 (1H, doublet, 8 Hz);
7.20 (1H, triplet, J = 8 Hz);
7.3 - 7.4 (5H, multiplet);
7.99 (2H, doublet, J = 8 Hz).

1(d) (3aR,4R,12aR,12bS) 4-Amino-2,3,3a,4,5, 12,12a,12b-octahydro-6-hydroxyindolo[3,2,1,-ij]benz[de]-quinolin-11(1H-one hydrochloride sesquihydrate

35 ml of a 1M solution of boron tribromide in methylene chloride were added to a suspension of 4.52 g of (3aR,4R,12aR,12bS) 4-benzyloxycarbonylamino-2,3,3a,4,5,12,12a,12b-octahydro-6-methoxyindolo-[3,2,1-ij]benz[de]quinolin-11(1 H )-one [prepared as described in step (c) above] in 35 ml of methylene chloride whilst ice-cooling, and the mixture was stirred at room temperature for 16 hours. At the end of this time, it was poured into 180 ml of a saturated aqueous solution of sodium hydrogencarbonate, and the mixture was stirred at room temperature for 30 minutes. The crystals which precipitated were collected by filtration, washed with water and then suspended in 35 ml of methanol. 3.5 ml of a 4N solution of hydrogen chloride in dioxane were then added to the suspension, and the resulting mixture was stirred at room temperature for 1 hour. At the end of this time, 70 ml of diisopropyl ether were added, and the mixture was stirred for 30 minutes. The crystals which precipitated were collected by filtration and reprecipitated from a mixture of methanol and ethyl acetate, to afford 3.32 g of the title compound, melting at 260 - 265° C (with decomposition).
Specific rotation: $[a]_D^{24}$ = +65.8° (c = 0.501, methanol).
Mass spectrum (m/e): 296 ($M^+$).

| Elemental analysis: | |
|---|---|
| Calculated for $C_{18}H_{20}N_2O_2$"HCP"3/2 $H_2O$: | |
| | C, 60.08%; H, 6.72%; N, 7.78%; CP, 9.85%. |
| Found: | C, 59.94%; H, 6.62%; N, 7.79%; CP, 9.87%. |

Infrared Absorption Spectrum (KBr) $n_{max}$ $cm^{-1}$ 3340, 1714.
Nuclear Magnetic Resonance Spectrum (hexadeuterated dimethyl sulphoxide) w ppm:
0.7 - 1.7 (6H, multiplet);
2.1 - 2.4 (2H, multiplet);
2.57 (1H, doublet of doublets, J = 2 & 17 Hz);
3.0 - 3.3 (5H, multiplet);
6.68 (1H, doublet, J = 8 Hz);
7.04 (1H, triplet, J = 8 Hz);
7.71 (1H, doublet, J = 8 Hz);
8.30 (3H, singlet);
9.81 (1H, singlet).

## EXAMPLE 2

(3aR,4R,12aR,12bS) 4-Amino-2,3,3a,4,5,12,12a,12b-octahydro-6-hydroxyindolo[3,2,1-ij]benz[de]quinolin-11-(1H)-one hydrochloride sesquihydrate

This Example illustrates the preparation of the same compound as was prepared in Example 1(d), but by a different route.

2(a) (R) p-Methoxybenzyl (E)-3-[4-benzyloxy-1-(2-cyclohexen-1-yl)acetyl-1H-indol-3-yl]propenoate

A procedure similar to that described in Example 1(a) was repeated, except that 4.73 g of p - methoxybenzyl ( E )-3-(4-benzyloxy-1 H -indol-3-yl)propenoate were reacted with 1.84 g of ( R )-2-cyclohexenylacetyl chloride, to give 5.2 g of the title compound, melting at 155 - 157 °C.
Nuclear Magnetic Resonance Spectrum ($CDCP_3$) w ppm:
1.3 - 1.8 (6H, multiplet);
1.9 - 2.1 (3H, multiplet);
3.81 (3H, singlet);
5.15 (2H, singlet);
5.25 (3H, singlet);
5.6 - 5.8 (2H, multiplet);
6.44 (1H, doublet, J = 16 Hz);
6.83 (1H, doublet, J = 8 Hz);
6.87 (1H, doublet, J = 8 Hz);
7.2 - 7.4 (6H, multiplet);
7.50 (1H, doublet, J = 8 Hz);
7.69 (1H, singlet);
8.11 (1H, doublet, J = 8 Hz);
8.35 (1H, doublets J = 16 Hz).
Infrared Absorption Spectrum (KBr) $n_{max}$ $cm^{-1}$:
1710, 1697.

2(b) (3aR,4R,12aR,12bS) 6-Benzyloxy-1,2,3,3a,4,5,11,12,12a,12b-decahydro-11-oxoindolo[3,2,1-ij]benz[de]-quinoline-4-carboxylic acid

3.50 g of ( R ) p -methoxybenzyl ( E )-3-[4-benzyloxy-1-(2-cyclohexen-1-yl)acetyl-1 H -indol-3-yl]-propenoate [prepared as described in step (a) above] were suspended in 35 ml of mesitylene, and the suspension was heated on an oil bath kept at 170° C for 25 hours. At the end of this time, the mixture was cooled, and 8.2 ml of a 4N solution of hydrogen chloride in dioxane were added to the reaction mixture. The mixture was then heated on an oil bath kept at 85° C for 4 hours, after which it was allowed to stand overnight at room temperature. The crystals which precipitated were collected by filtration and washed with diethyl ether, to afford 2.02 g of the title compound, melting at 269 - 271° C (after recrystallization from a mixture of ethanol and chloroform).

Specific rotation: $[a]_D^{23}$ = +89.4° (c = 0.507, dimethyl sulphoxide).

Mass spectrum (m/e): 415 ($M^+$).

| Elemental analysis: | |
|---|---|
| Calculated for $C_{26}H_{25}NO_4$: | |
| | C, 75.16%; H, 6.07%; N, 3.37%. |
| Found: | C, 75.20%; H, 6.02%; N, 3.55%. |

Infrared Absorption Spectrum (KBr) $n_{max}$ $cm^{-1}$:
1732, 1700, 1673.

Nuclear Magnetic Resonance Spectrum (hexadeuterated dimethyl sulphoxide) w ppm:
0.6 - 1.7 (6H, multiplet);
2.1 - 2.3 (1H, multiplet);
2.4 - 2.6 (2H, multiplet);
2.7 - 2.9 (2H, multiplet);
3.0 - 3.4 (3H, multiplet);
5.20 (2H, singlet);
6.91 (1H, doublet, J = 8 Hz);
7.16 (1H, triplet, J = 8 Hz);
7.3 - 7.6 (5H, multiplet);
7.84 (1H, doublet, J = 8 Hz);
12.4 (1H, broad singlet).

2(c) (3aR,4R,12aR,12bS) 6-Benzyloxy-4-benzyloxy-carbonylamino-2,3,3a,4,5,12,12a,12b-octahydroindolo-[3,2,1-ij]benz[de]quinolin-11(1H)-one

Following a procedure similar to that described in Example 1(c), 402 mg of the title compound were obtained from 830 mg of (3a R ,4 R ,12a R ,12b S ) 6-benzyloxy-1,2,3,3a,4,5,11,12,12a,12b-decahydro-1 1-oxoindolo-[3,2,1,-ij]benz[de]quinoline-4-carboxylic acid [prepared as desoribed in step (b) above].

Specific rotation: $[a]_D^{20}$ = 90.0° (c = 1.00, $CHCP_3$).

Nuclear Magnetic Resonance Spectrum ($CDCP_3$) w ppm:
0.8 - 1.8 (6H, multiplet);
2.1 - 2.4 (2H, multiplet);
2.67 (1H, doublet of doublets, J = 2 & 17 Hz);
2.9 - 3.0 (2H, multiplet);
3.1 - 3.25 (2H, multiplet);
4.0 - 4.1 (1H, multiplet);
5.0 - 5.2 (2H, multiplet);
5.15 (2H, singlet);
6.78 (1H, doublet, J = 8 Hz);
7.20 (1H, triplet, J = 8 Hz);
7.2 - 7.5 (10H, multiplet);
8.02 (1H, doublet, J = 8 Hz).

2(d) (3aR,4R,12aR,12bS) 4-Amino-2,3,3a,4,5,12,12a,12b-octahydro-6-hydroxyindolo[3,2,1-ij]benz[de]quinolin-11(1H)-one hydrochloride sesquihydrate

402 mg of (3a R ,4 R ,12a R ,12b S ) 6-benzyloxy-4-benzyloxycarbonylamino-2,3,3a,4,5,12,12a,12b-octahydroindolo[3,2,1-ij]benz[de]quinolin-11(1 H )-one [prepared as described in step (c) above] were suspended in a mixture of 10 ml of ethanol and 10 ml of water. The suspension was vigorously stirred at room temperature under an atmosphere of hydrogen and in the presence of 0.25 ml of concentrated hydrochloric acid and 400 mg of 10% w/w palladium-on-charcoal for 2 hours. At the end of this time, the catalyst was removed by filtration, the solvent was removed from the filtrate by distillation under reduced pressure, and the residue was triturated with a small amount of ethanol to precipitate crystals. These crystals were collected by filtration and washed with ethanol, to afford 252 mg of the title compound.

The properties of this product were identical to those of the product of Example 1(d), and this product can be used in Example 6 in place of the product of Example 1(d).

## EXAMPLE 3

### (3aR,4R,12aR,12bS) 2,3,3a,4,5,12,12a,12b-Octahydro-6-hydroxy-4-(pyrrolidin-1-yl)indolo[3,2,1-ij]benz[de]quinoline-11(1H)-one hydrochloride

3(a) (3aR,4R,12aR,12bS) 4-Amino-2,3,3a,4,5,12,12a,12b-octahydro-6-methoxyindolo[3,2,1-ij]benz[de]quinolin-11(1H)-one monohydrate

9.60 g of (3a R ,4 R ,12a R ,12b S ) 4-benzyloxycarbonylamino-2,3,3a,4,5,12,12a,12b-octahydro-6-methoxyindolo[3,2,1-ij]benz[de]quinolin-11(1 H )-one [prepared as described in Example 1(c)] were dissolved in 200 ml of tetrahydrofuran. The solution was then stirred at room temperature under an atmosphere of hydrogen and in the presence of 2.5 g of 10% w/w palladium-on-charcoal for 7 hours. At the end of this time, methanol was added, in order to dissolve the crystals which precipitated. The catalyst was removed by filtration, and the solvent was removed from the filtrate by evaporation under reduced pressure. The residue was triturated with ethanol, and the resulting crystals were collected by filtration to afford 5.33 g of the title compound as crystals, melting at 77 - 79 °C.
Mass spectrum (m/e): 310 ($M^+$).

| Elemental analysis: | |
|---|---|
| Calculated for $C_{19}H_{22}N_2O_2$"$H_2O$: | |
| | C, 69.49%; H, 7.37%; N, 8.53%. |
| Found: | C, 69.24%; H, 7.30%; N, 8.35%. |

Infrared Absorption Spectrum (KBr) $n_{max}$ $cm^{-1}$:
3350, 1704.
Nuclear Magnetic Resonance Spectrum (hexadeuterated dimethyl sulphoxide) w ppm:
0.6 - 1.6 (6H, multiplet);
1.8 - 2.2 (2H, multiplet);
2.51 (1H, doublet of doublets, J = 2 & 17 Hz);
2.69 (1H, doublet, J = 18 Hz);
2.81 (1H, doublet of triplets, J = 4 & 17 Hz);
3.11 (1H, doublet of doublets, J = 5 & 17 Hz);
3.1 - 3.4 (2H, multiplet);
6.78 (1H, doublet, J = 8 Hz);
7.14 (1H, triplet, J = 8 Hz);
7.84 (1H, doublet, J = 8 Hz).

3(b) (3aR,4R,12aR,12bS) 2,3,3a,4,5,12,12a,12b-Octahydro-6-methoxy-4-(pyrrolidin-1-yl)indolo[3,2,1-ij]benz-[de]quinolin-11(1H)-one

403 mg of sodium hydrogencarbonate were added to a solution of 500 mg of (3a R ,4 R ,12a R ,12b S ) 4-amino-2,3,3a,4,5,12,12a,12b-octahydro-6-methoxyindolo[3,2,1-ij]benz[de]quinolin-11(1 H )-one [prepared as described in step (a) above] and 1.48 g of 1,4-diiodobutane in 30 ml of benzene, and the mixture was heated under reflux for 19 hours. At the end of this time, the reaction mixture was diluted with ethyl acetate and washed with water and with a saturated aqueous solution of sodium chloride, in that order. The solvent was then removed by distillation under reduced pressure, and then the residue was purified by column chromatography through silica gel using a 100 : 10 : 1 by volume mixture of methylene chloride, ethanol and aqueous ammonia as the eluent to afford 343 mg of the title compound as crystals, melting at 184 - 187 ° C.
Mass spectrum (m/e): 364 ($M^+$).
Infrared Absorption Spectrum (KBr) $n_{max}$ $cm^{-1}$:
1702.
Nuclear Magnetic Resonance Spectrum ($CDCP_3$) w ppm:
0.9 - 1.9 (10H, multiplet);
2.1 - 2.4 (2H, multiplet);
2.4 - 2.5 (1H, multiplet);
2.62 (1H, doublet of doublets, J = 2 & 17 Hz);
2.6 - 2.8 (4H, multiplet);
2.81 (1H, doublet of triplets, J = 4 & 19 Hz);
2.95 (1H, doublet of doublets, J = 5 & 17 Hz);
3.26 (1H, doublet, J = 19 Hz);
3.25 - 3.35 (1H, multiplet);
3.88 (3H, singlet);
6.68 (1H, doublet, J = 8 Hz);
7.15 (1H, triplet, J = 8 Hz);
7.98 (1H, doublet, J = 8 Hz).

3(c) (3aR,4R,12aR,12bS) 2,3,3a,4,5,12,12a,12b-Octahydro-6-hydroxy-4-(pyrrolidin-1-yl)indolo[3,2,1-ij]benz-[de]quinolin-11(1H-one hydrochloride

470 mg of (3a R ,4 R ,12a R ,12b S ) 2,3,3a,4,5,12,12a,12b-octahydro-6-methoxy-4-(pyrrolidin-1-yl)-indolo[3,2,1-ij]-benz[de]quinolin-11(1 H )-one [prepared as described in step (b) above] were dissolved in 10 ml of a 4N solution of hydrogen chloride in dioxane, and the solution was stirred for a short while. The solvent was then removed by distillation under reduced pressure, to afford a hydrochloride. 4 ml of a 1M solution of boron tribromide in methylene chloride were added to the hydrochloride whilst ice-cooling, and the mixture was stirred overnight at room temperature. At the end of this time, a saturated aqueous solution of sodium hydrogencarbonate was added, and the mixture was extracted with methylene chloride. The extract was washed with water and dried over anhydrous sodium sulphate, after which the solvent was removed by evaporation under reduced pressure. The residue was triturated with ethanol to afford 162 mg of the title compound as crystals, melting at 235 - 240 ° C.
Mass spectrum (m/e): 350 ($M^+$).
Infrared Absorption Spectrum (KBr) $n_{max}$ $cm^{-1}$:
3460, 1695.
Nuclear Magnetic Resonance Spectrum (hexadeuterated dimethyl sulphoxide) w ppm:
0.7 - 1.6 (6H, multiplet);
1.8 - 2.1 (4H, multiplet);
2.2 - 2.4 (1H, multiplet);
2.5 - 2.6 (2H, multiplet);
3.02 (1H doublet of doublets, J = 5 & 17 Hz);
3.1 - 3.4 (5H, multiplet);
3.6 - 3.8 (3H, multiplet);
6.69 (1H, doublet, J = 8 Hz);
7.05 (1H, triplet, J = 8 Hz);
7.71 (1H, doublet, J = 8 Hz);

9.80 (1H, singlet);
9.22 (1H, singlet);

## EXAMPLE 4

### (3aR,4R,12aR,12bS) 2,3,3a,4,5,12,12a,12b-Octahydro-6-hydroxy-4-bis(2-hydroxyethyl)aminoindolo[3,2,1-ij]-benz-[de]quinolin-11(1H)-one hydrochloride

#### 4(a) (3aR,4R,12aR,12bS) 2,3,3a,4,5,12,12a,12b-Octahydro-4-bis(2-hydroxyethyl)amino-6-methoxyindolo-[3,2,1-ij]benz[de]quinolin-11(1H)-one

Following a procedure similar to that described in Example 3(b), but using 500 mg of (3a R ,4 R ,12a R ,12b S ) 4-amino-2,3,3a,4,5,12,12a,12b-octahydro-6-methoxyindolo[3,2,1-ij]benz[de]quinolin-11(1 H )-one [prepared as described in Example 3(a)] and 1.25 ml of 2-iodoethanol, 400 mg of the title compound were obtained as crystals, melting at 184 - 188° C.

Mass spectrum (m/e): 398 ($M^+$).
Infrared Absorption Spectrum (KBr) $n_{max}$ $cm^{-1}$:
3410, 1700.
Nuclear Magnetic Resonance Spectrum ($CDCP_3$) w ppm:
0.8 - 1.8 (6H, multiplet);
2.1 - 2.3 (2H, multiplet);
2.3 - 2.7 (2H, broad singlet);
2.65 (1H, doublet of doublets, J = 2 & 17 Hz);
2.84 (4H, triplet, J = 5.5 Hz);
2.97 (1H, doublet of doublets, J = 5 & 17 Hz);
3.01 - 3.15 (3H, multiplet);
3.25 - 3.35 (1H, multiplet);
3.59 (4H, triplet, J = 5.5 Hz);
3.89 (3H, singlet);
6.71 (1H, doublet, J = 8 Hz);
7.20 (1H, triplet, J = 8 Hz);
8.02 (1H, doublet, J = 8 Hz).

#### 4(b) (3aR,4R,12aR,12bS) 2,3,3a,4,5,12,12a,12b-Octahydro-6-hydroxy-4-bis(2-hydroxyethyl)aminoindolo-[3,2,1-ij]benz[de]quinolin-11(1H)-one hydrochloride

Following a procedure similar to that described in Example 3(c), but using 383 mg of (3a R ,4 R ,12a R ,12b S ) 2,3,3a,4,5,12,12a,12b-octahydro-4-bis(2-hydroxyethyl)amino-6-methoxyindolo[3,2,1-ij]benz[de]-quinolin-11(1 H )-one [prepared as described in step (a) above], 184 mg of the title compound were obtained as crystals, melting at 140 - 145° C (with decomposition).

Mass spectrum (m/e): 384 ($M^+$ - HCP).
Infrared Absorption Spectrum (KBr) $n_{max}$ $cm^{-1}$:
3230, 1703.
Nuclear Magnetic Resonance Spectrum (hexadeuterated dimethyl sulphoxide) w ppm:
0.6 - 1.7 (6H, multiplet);
2.20 - 2.35 (1H, multiplet);
2.5 - 2.6 (1H, multiplet);
2.7 - 2.8 (1H, multiplet);
3.07 (1H, doublet of doublets, J = 5 & 17 Hz);
3.30 - 3.95 (12H, multiplet);
5.3 - 5.5 (2H, multiplet);

6.68 (1H, doublet, J = 8 Hz);
7.05 (1H, triplet, J = 8 Hz);
7.71 (1H, doublet, J = 8 Hz);
9.30 (1H, broad singlet);
9.81 (1H, singlet).

EXAMPLE 5

(3aR,4R,12aR,12bS) 4-Dimethylamino-2,3,3a,4,5,12,12a,12b-octahydro-6-hydroxyindolo[3,2,1-ij]benz[de]quinolin-11(1H)-one hydrochloride

5(a) (3aR,4R,12aR,12bS) 4-Dimethylamino-2,3,3a,4,5,-12,12a,12b-octahydro-6-methoxyindolo[3,2,1-ij]benz-[de]quinolin-11(1H)-one

A mixture of 500 mg of (3a R ,4 R ,12a R ,12b S ) 4-amino-2,3,3a,4,5,12,12a,12b-octahydro-6-methoxyindolo[3,2,1-ij]benz[de]quinolin-11(1 H )-one [prepared as described in Example 3(a)], 653 mg of formalin (37% aqueous formaldehyde) and 0.298 ml of formic acid was heated on an oil bath at 100° C for 2 hours. At the end of this time, the reaction mixture was poured into a saturated aqueous solution of sodium hydrogencarbonate and extracted with methylene chloride. The extract was washed with a saturated aqueous solution of sodium hydrogencarbonate and water, in that order, and dried over anhydrous sodium sulphate. The solvent was then removed by distillation under reduced pressure, and the residue was purified by column chromatography through silica gel eluted with a 100 : 10 : 1 by volume mixture of methylene chloride, ethanol and aqueous ammonia to give 227 mg of the title compound as an amorphous substance.
Mass spectrum (m/e): 338 ($M^+$).
Infrared Absorption Spectrum (KBr) $n_{max}$ $cm^{-1}$:
1703.
Nuclear Magnetic Resonance Spectrum ($CDCP_3$) w ppm:
0.8 - 1.8 (6H, multiplet);
2.1 - 2.5 (3H, multiplet);
2.40 (6H, singlet);
2.62 (1H, doublet of doublets J = 2 & 17 Hz);
2.77 (1H, doublet of triplets, J = 4 & 19 Hz);
2.95 (1H, doublet of doublets, J = 5 & 17 Hz);
3.2 - 3.3 (1H, multiplet);
3.32 (1H, doublet, J = 19 Hz);
3.89 (3H, singlet);
6.68 (1H, doublet, J = 8 Hz);
7.16 (1H, triplet, J = 8 Hz);
7.98 (1H, doublet, J = 8 Hz).

5(b) (3aR,4R,12aR,12bS) 4-Dimethylamino-2,3,3a,4,5,12,12a,12b-octahydro-6-hydroxyindolo[3,2,1-ij]benz-[de]quinolin-11(1H)-one hydrochloride

A procedure similar to that described in Example 3(C) was repeated, except that 220 mg of (3a R ,4 R ,12a R ,12b S ) 4-dimethylamino-2,3,3a,4,5,12,-12a,12b-octahydro-6-methoxyindolo[3,2,1-ij]benz[de]-quinolin-11(1 H )-one [prepared as described in step (a) above] and 4 ml of a 1 M solution of boron tribromide in methylene chloride were employed, to give 163 mg of the title compound as crystals, melting at 215 - 217° C.
Mass spectrum (m/e): 324 ($M^+$-HCP).
Infrared Absorption Spectrum (KBr) $n_{max}$ $cm^{-1}$:
3200, 1697.

Nuclear Magnetic Resonance Spectrum ($CD_3OD$) w ppm:
0.8 - 1.8 (6H, multiplet);
2.3 - 2.4 (1H, multiplet);
2.63 (1H, doublet of doublets, J = 2 & 17 Hz);
2.6 - 2.8 (1H, multiplet);
3.03 (6H, singlet);
3.09 (1H, doublet of doublets, J = 5 & 17 Hz);
3.2 - 3.4 (2H, multiplet);
3.5 - 3.7 (2H, multiplet);
6.65 (1H, doublet, J = 8 Hz);
7.08 (1H, triplet, J = 8 Hz);
7.79 (1H, doublet, J = 8 Hz).

## EXAMPLE 6

### (3aR,4R,12aR,12bS) 4-Ethylamino-6-hydroxy-2,3,3a,4,5,12,12a,12b-octahydroindolo[3,2,1-ij]benz[de]quinoline-11-(1H)-one hydrochloride

6(a) (3aR,4R,12aR,12bs) 4-Amino-6-benzyloxy-2,3,3a,4,5,12,12a,12b-octahydroindolo[3,2,1-ij]benz[de]-quinolin-11(1H)-one

31 mg of sodium hydride (as a 55% by weight suspension in mineral oil) and 191 mg of benzyl chloride were added under a nitrogen atmosphere to a solution of 323 mg of (3a R ,4 R ,12a R ,12b S ) 4-amino-2,3,3a,4,5,12,12a,12b-octahydro-6-hydroxyindolo[3,2,1-ij]benz[de]quinolin-11(1 H )-one [prepared as described in Example 1(d)] in 3 ml of dimethylformamide, and the mixture was stirred at room temperature overnight. The reaction mixture was then poured into water and extracted with methylene chloride. The extract was washed with water and then dried over anhydrous magnesium sulphate, after which the solvent was removed by distillation under reduced pressure. The residue was purified by column chromatography through silica gel eluted with methanol to give 120 mg of the title compound.
Infrared Absorption Spectrum (liquid film) $n_{max}$ $cm^{-1}$:
1700, 3380.
Nuclear Magnetic Resonance Spectrum ($CDCP_3$) w ppm:
0.6 - 3.5 (16H, multiplet);
5.09 (2H, singlet);
6.71 (1H, doublet, J = 8 Hz);
7.13 (1H, triplet, J = 8 Hz);
7.2 - 7.6 (5H, multiplet);
8.02 (1H, doublet, J = 8 Hz).

6(b) (3aR,4R,12aR,12bS) 6-Benzyloxy-4-ethylamino-2,3,3a,4,5,12,12a,12b-octahydroindolo[3,2,1-ij]benz[de]-quinolin-11(1H)-one

Following a procedure similar to that described in Example 3(b), 320 mg of the title compound were obtained as an amorphous substance from 450 mg of (3a R ,4 R ,12a R ,12b S ) 4-amino-6-benzyloxy-2,3,3a,4,5,12,12a,12b-octahydroindolo[3,2,1-ij]benz[de]quinolin-11(1 H -one [prepared as described in step (a) above] and 0.4 ml of ethyl iodide.
Nuclear Magnetic Resonance Spectrum ($CDCP_3$) w ppm:
0.7 - 3.3 (15H, multiplet);
1.10 (3H, triplet, J = 7 Hz);
2.73 (2H, quartet, J = 7 Hz);
5.11 (2H, singlet);
6.71 (1H, doublet, J = 8 Hz);

7.12 (1H, triplet, J = 8 Hz);
7.2 - 7.6 (5H, multiplet);
8.00 (1H, doublet, J = 8 Hz).

6(c) (3aR,4R,12aR,12bs) 4-Ethylamino-6-hydroxy-2,3,3a,4,5,12,12a,12b-octahydroindolo[3,2,1-ij]benz[de]-quinolin-11(1H)-one hydrochloride

0.25 ml of concentrated hydrochloric acid and 300 mg of 10% w/w palladium-on-carbon were added to a suspension of 320 mg of (3a R ,4 R ,12a R ,12b S ) 6-benzyloxy-4-ethylamino-2,3,3a,4,5,12,12a,12b-octahydroindolo[3,2,1-ij]benz[de]quinolin-11(1 H )-one [prepared as described in step (b) above] in 10 ml of ethanol and 10 ml of water, and the mixture was stirred at room temperature for 3 hours under a hydrogen atmosphere. At the end of this time, the catalyst was removed by filtration and the filtrate was evaporated under reduced pressure. The residue was dissolved in ethanol and reprecipitated by the addition of diisopropyl ether, to give 205 mg of the title compound, melting at 255 - 260° C (with decomposition).
Mass spectrum (m/e): 324 ($M^+$-HCP).
Infrared Absorption Spectrum (KBr) $n_{max}$ $cm^{-1}$
1704.
Nuclear Magnetic Resonance Spectrum (hexadeuterated dimethyl sulphoxide) w ppm:
0.6 - 1.7 [6H, multiplet);
1.26 (3H, triplet, J = 7 Hz);
2.1 - 2.3 (1H, multiplet);
2.4 - 2.6 (2H, multiplet);
2.9 - 3.3 (6H, multiplet);
3.5 - 3.6 (1H, multiplet);
6.69 (1H, doublet, J = 8 Hz);
7.05 (1H, triplet, J = 8 Hz);
7.70 (1H, doublet, J = 8 Hz);
8.80 (2H, broad);
9.81 (1H, singlet).

## PREPARATION 1

### (R)-2-Cyclohexen-1-ylacetic acid

806 mg of o -nitrophenol were added to a mixture of 19.0 g of ( R )-2-cyclohexen-1-ol of optical purity 79% ee [prepared by a procedure similar to that described in Chemistry Letters, 829 (1984)] and 100 ml of ethyl orthoacetate. The resulting mixture was heated on an oil bath kept at 170° C for 9 hours and the ethanol formed in the course of the reaction was removed by distillation. After removal of excess ethyl orthoacetate by evaporation under reduced pressure, 22.9 g of ethyl ( R )-2-cyclohexenylacetate were obtained by fractional distillation, boiling at 50 - 52° C/0.4 mmHg (53 Pa).

200 ml of a solution of 11.2 g of sodium hydroxide in a 4 : 1 by volume mixture of methanol and water were added to the whole of this ester, and the mixture was heated on an oil bath kept at 85° C for 3 hours. At the end of this time, the mixture was concentrated by distillation under reduced pressure, to remove the methanol. The residue was mixed with 100 ml of water and washed with diethyl ether. The aqueous layer was adjusted to a pH value of 2 by the addition of 10% v/v aqueous hydrochloric acid and then extracted with diethyl ether. The extract was dried over anhydrous magnesium sulphate, and the solvent was removed by evaporation under reduced pressure. 15.4 g of the title compound were obtained by fractional distillation, boiling at 89 - 91° C/0.6mmHg (80 Pa).
Specific rotation: $[a]_D^{25}$ = -51.1° (c = 0.52, methanol).
Nuclear Magnetic Resonance Spectrum ($CDCP_3$) w ppm:
1.0 - 2. 9 (9H, multiplet);
5.4 - 5.9 (2H, multiplet);
11.75 (1H, singlet).

PREPARATION 2

(R)-2-Cyclohexen-1-ylacetyl chloride

11.97 ml of thionyl chloride were added to a solution of 15. 3 g of ( R )-2-cyclohexen-1-ylacetic acid (prepared as described in Preparation 1) in 45 ml of benzene, and the mixture was heated on an oil bath kept at 90° C for 1.5 hours. At the end of this time, the excess of thionyl chloride and benzene were distilled off under reduced pressure, and 16.3 g of the title compound were obtained by fractional distillation as a colourless oily liquid boiling at 69 - 71 ° C/3.0 mmHg (400 Pa).
Nuclear Magnetic Resonance Spectrum ($CDCP_3$) w ppm:
1.1 - 2.2 (6H, multiplet);
2.4 - 3.1 (3H, multiplet);
5.3 - 6.0 (2H, multiplet).

PREPARATION 3

(1R,2R)-2-Phenylthio-1-cyclohexyl acetate

4.4 ml of isopropenyl acetate and 10 g of Lipase PS (Amano Pharmaceutical Industry) were added to a solution of 4.17 g of trans -2-phenylthiocyclohexanol (racemate) in 50 ml of diisopropyl ether, and the mixture was stirred on a water bath at 23° C for 15 hours. At the end of this time, the Lipase was removed by filtration and the filtrate was evaporated under reduced pressure. The residue was purified by column chromatography through silica gel eluted with a 50 : 1 by volume mixture of cyclohexane and ethyl acetate, to give two oily substances.

Distillation of the less polar substance gave 2.33 g of the title compound as a colourless oil, boiling at 153° C/1.8 mmHg (240 Pa).
Specific Rotation $[a]_D^{23}$ = +6.88° (c = 1.25, $CHCP_3$).
Mass spectrum (m/e): 250 ($M^+$).
Infrared Absorption Spectrum ($CHCP_3$) $n_{max}$ $cm^{-1}$:
1730.
Nuclear Magnetic Resonance Spectrum ($CDCP_3$) w ppm:
1.2 - 2. 2 (8H, multiplet);
3.1 - 3.2 (1H, multiplet);
4.25 - 4.35 (1H, multiplet);
7.2 - 7.5 (5H, multiplet);
1.92 (3H, singlet).

Distillation of the more polar substance gave 1.88 g of (1 S ,2 S )-2-phenylthiocyclohexanol as a colourless oil, boiling at 137° C/1.5 mmHg (200 Pa).
Mass spectrum (m/e): 208 ($M^+$).
Specific Rotation $[a]_D^{23}$ = +71.9° (c = 1.21, $CHCP_3$).
Infrared Absorption Spectrum ($CHCP_3$) $n_{max}$ $cm^{-1}$:
3540.
Nuclear Magnetic Resonance Spectrum ($CDCP_3$) w ppm:
1.2 - 2.2 (8H, multiplet);
2.7 - 2.85 (1H, multiplet);
3.25 - 3.4 (1H, multiplet);
7.25 - 7.5 (5H, multiplet).

The optical purities of the above two compounds were determined by high performance liquid chromatography (column: Chiralcel OD, Daiseru Chemical, solvent: a 97 : 3 by volume mixture of hexane and isopropanol and a flow rate of solvent: 1 ml/min); their purities were found to be over 99% ee.

PREPARATIONS 4 TO 10

(1R,2R)-2-Phenylthio-1-cyclohexyl acetate

The same reactions as described in Preparation 3 were carried out in various solvents as listed in the following Table 2, except that the stirring was effected for 24 hours, and the results are summarized in Table 2.

Table 2

| Prep No. | Solvent | Conversion of 2-phenylthiocyclohexanol (racemate) (%)*) |
|---|---|---|
| 4 | diisopropyl ether | 50 |
| 5 | hexane | 50 |
| 6 | acetonitrile | 49 |
| 7 | acetone | 50 |
| 8 | tetrahydrofuran | 50 |
| 9 | dioxane | 45 |
| 10 | benzene | 38 |

*) Only unreacted 2-phenylthiocyclohexanol and the title compound were detected.

The optical purities of the title compound in these Preparations were over 99% ee.

PREPARATION 11

(1R, 2R)-2-Phenylthio-1-cyclohexyl acetate

A procedure similar to that described in Preparation 3 was repeated except that acetic anhydride was employed in place of isopropenyl acetate and the mixture was stirred for 24 hours. Conversion of the reagent was 49.8% and the optical purity of the title compound was 99% ee.

PREPARATION 12

(R)-2-Cyclohexen-1-yl acetate

2.19 g of sodium periodate were added to a mixture of 2.33 g of (1 R ,2 R )-2-phenylthio-1-cyclohexyl acetate (prepared as described in Preparation 3) in 46 ml of 50% by volume aqueous methanol and the mixture was stirred at room temperature overnight. At the end of this time, insoluble material was removed by filtration, the methanol was removed by evaporation under reduced pressure, and the residue was extraoted with ethyl acetate. The extract was washed with a saturated aqueous solution of sodium hydrogencarbonate and with a saturated aqueous solution of sodium chloride, in that order, after which it was dried over anhydrous sodium sulphate. The solvent was then removed by evaporation under reduced

pressure, and 1.3 g of calcium carbonate were added to the residue. The mixture was then heated on an oil bath at 150° C under reduced pressure (20 mmHg - 2666 Pa) for 3.5 hours and the product was distilled off. Further distillation of the product gave 0.953 g of the title compound, boiling at 77° C/20 mmHg (2666 Pa).
Specific Rotation $[a]_D^{23}$ = +205.47° (c = 1.61, $CHCP_3$).
Mass spectrum (m/e): 140 ($M^+$).
Infrared Absorption Spectrum (liquid film) $n_{max}$ $cm^{-1}$:
1725.
Nuclear Magnetic Resonance Spectrum ($CDCP_3$) w ppm:
1.5 - 2.2 (6H, multiplet);
2.05 (3H, singlet);
5.2 - 5.3 (1H, multiplet);
5.15 - 5.25 (1H, multiplet);
5.9 - 6.0 (1H, multiplet).

## PREPARATION 13

## (R)-2-Cyclohexen-1-ol

A mixture of 0.920 g of ( R )-2-cyclohexen-1-yl acetate (prepared as described in Preparation 12) and 0.476 g of potassium hydroxide in 20 ml of methanol was stirred at room temperature overnight. At the end of this time, the methanol was removed by evaporation under reduced pressure, water was added to the residue and the mixture was extracted with diethyl ether. The extract was washed with a saturated aqueous solution of sodium chloride and dried over anhydrous sodium sulphate. The solvent was removed by evaporation under reduced pressure, and the resulting residue was distilled to give 0.42 g of the title compound as a colourless oil, boiling at 83° C/35 mmHg (4666 Pa).
Specific Rotation $[a]_D^{23}$ = +130.58° (c = 1.21, $CHCP_3$).
Nuclear Magnetic Resonance Spectrum ($CDCP_3$) w ppm
1.4 - 2.4 (7H, multiplet);
4.0 - 4.3 (1H, multiplet);
5.5 - 6.0 (2H, multiplet).

**Claims**

1. Optically active compounds of formula (I):

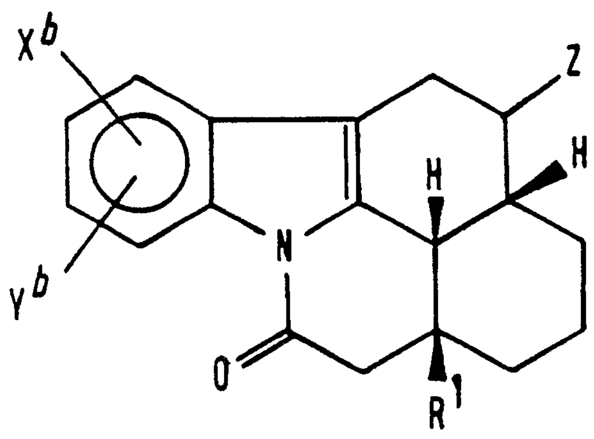

(I)

in which:
$R^1$ represents a hydrogen atom or an alkyl group having from 1 to 4 carbon atoms;
$X^b$ and $Y^b$ are the same or different and each represents a hydrogen atom or a hydroxy group; and

Z represents a group of formula $-NR^aR^b$
in which $R^a$ and $R^b$ are the same or different and each represents a hydrogen atom, an alkyl group having from 1 to 4 carbon atoms or a hydroxyalkyl group having at least one hydroxy group and having from 1 to 4 carbon atoms,
or a cyclic amino group having from 3 to 6 ring atoms, of which 1 or 2 are nitrogen atoms, 0 or 1 is an oxygen or sulphur atom and the remainder is or are carbon atoms;
and pharmaceutically acceptable salts thereof.

2. A compound according to Claim 1, in whioh the group represented by Z is in the R-configuration.

3. A compound according to Claim 1 or Claim 2, in which $R^1$ represents a hydrogen atom, a methyl group or an ethyl group.

4. A compound according to Claim 1 or Claim 2, in which $R^1$ represents a hydrogen atom.

5. A compound according to any one of Claims 1 to 4, in which one of $X^b$ and $Y^b$ represents a hydroxy group and the other represents a hydrogen atom.

6. A compound according to any one of Claims 1 to 5, in which Z represents an amino, ethylamino, dimethylamino, diethylamino, pyrrolidyl or piperidyl group.

7. A compound according to any one of Claims 1 to 5, in which Z represents an amino, ethylamino or dimethylamino group.

8. A compound according to any one of Claims 1 to 6, in which $X^b$ represents a hydroxy group at the 6- or 7-position and $Y^b$ represents a hydrogen atom.

9. A compound according to Claim 8, in whioh $X^b$ represents a hydroxy group at the 6-position.

10. A compound according to Claim 1 or Claim 2, in which:
$R^1$ represents a hydrogen atom, a methyl group or an ethyl group;
one of $X^b$ and $Y^b$ represents a hydroxy group and the other represents a hydrogen atom; and
Z represents an amino, ethylamino, dimethylamino, diethylamino, pyrrolidyl or piperidyl group.

11 A compound according to Claim 1 or Claim 2, in which:
$R^1$ represents a hydrogen atom;
one of $X^b$ and $Y^b$ represents a hydroxy group and the other represents a hydrogen atom; and
Z represents an amino, ethylamino or dimethylamino group.

12. A compound according to Claim 11, in which $X^b$ represents a hydroxy group at the 6-position.

13. (3a R ,4 R , 12a R , 12b S ) 4-Amino-2,3,3a,4,5,12,12a,12b-octahydro-6-hydroxyindolo[3,2,1,-ij]benz[de]-quinolin-11(1 H )-one and pharmaceutically acceptable salts thereof.

14. (3a R ,4 R ,12a R ,12b S ) 4-Amino-2,3,3a,4,5,12,12a,12b-octahydro-6-hydroxy-12a-methylindolo[3,2,1,-ij]benz[de]quinolin-11(1 H -one and pharmaceutically acceptable salts thereof.

15. (3a R ,4 R , 12a R , 12b S ) 4-Amino-12a-ethyl-2,3,3a,4,5,12,12a,12b-octahydro-6-hydroxyindolo[3,2,1,-ij]benz[de]quinolin-11(1 H )-one and pharmaceutically acceptable salts thereof.

16. (3a R ,4 R ,12a R ,12b S ) 4-(Dimethylamino)-2,3,3a,4,5,12,12a,12b-octahydro-6-hydroxyindolo[3,2,1,-ij]-benz[de] quinolin-11(1 H )-one and pharmaceutically acceptable salts thereof.

17. (3a R ,4 R ,12a R ,12b S ) 4-Ethylamino-2,3,3a,4,5,12,12a,12b-octahydro-6-hydroxyindolo[3,2,1,-ij]benz-[de]quinolin-11(1 H )-one and pharmaceutically acceptable salts thereof.

18. (3a R ,4 R ,12a R ,12b S ) 2,3,3a,4,5,12,12a,12b-Octahydro-6-hydroxy-4-pyrrolidin-1-ylindolo-[3,2,1,-ij]-benz[de]quinolin-11(1 H )-one and pharmaceutically acceptable salts thereof.

19. (3a R ,4 R ,12a R ,12b S ) 2,3,3a,4,5,12,12a,12b-Octahydro-6-hydroxy-4-bis(2-hydroxyethyl)aminoindolo-[3,2,1-ij]benz[de]quinolin-11(1 H )-one and pharmaceutically acceptable salts thereof.

20. A pharmaceutical composition for the treatment or prophylaxis of arrhythmia, which comprises an anti-arrhythmic compound in admixture with a pharmaceutically acceptable carrier or diluent, in which the anti-arrhythmic compound is at least one optically active compound of formula (I) or a pharmaceutically acceptable salt thereof, as defined in any one of Claims 1 to 19.

21. The use of at least one optically active compound of formula (I) or a pharmaceutically acceptable salt thereof, as defined in any one of Claims 1 to 19, for the treatment or prophylaxis of arrhythmia in a mammal

22. The use of at least one optically active compound of formula (I) or a pharmaceutically acceptable salt thereof, as defined in any one of Claims 1 to 19, for the manufacture of a medicament for the treatment or prophylaxis of arrhythmia in a mammal

23. A process for preparing an optically active compound according to any one of Claims 1 to 19, which process comprises the steps:

(a) heating a compound of formula (IV)

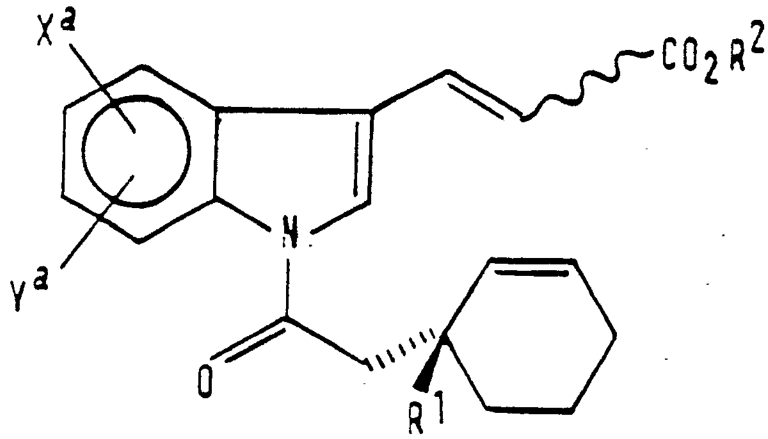

(IV)

(in which: $R^1$ is as defined in Claim 1; $R^2$ represents a carboxy-protecting group; and $X^a$ and $Y^a$ are the same or different and each represents a hydrogen atoms and protected hydroxy groups) to afford a compound of formula (V):

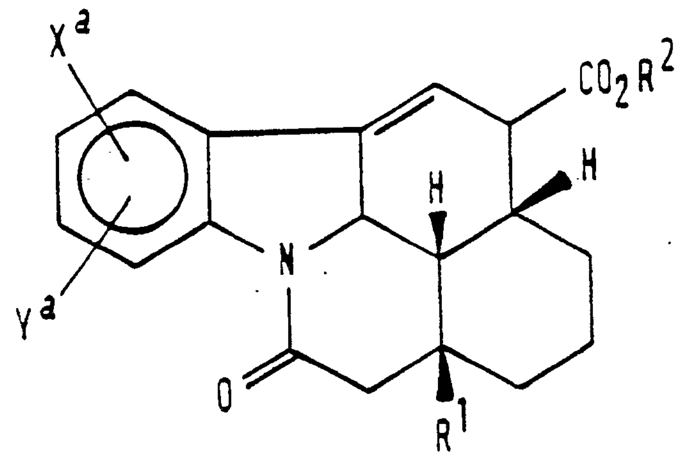

(V)

(in which $R^1$, $R^2$, $X^a$ and $Y^a$ are as defined above);

(b) isomerizing the compound of formula (V) and removing the carboxy-protecting group, to give a compound of formula (VI):

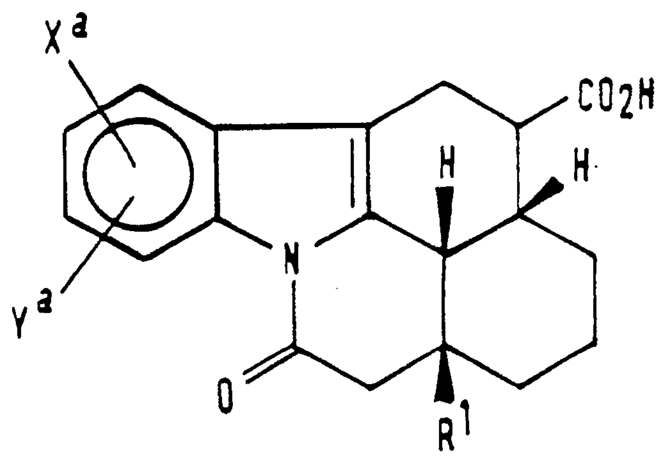

(VI)

(in which $R^1$, $X^a$ and $Y^a$ are as defined above);

(c) azidating and heating the compound of formula (VI), and then reacting it with an alcoholic compound

of formula (IX):

$R^3OH$ (IX)

(in which $R^3$ represents an alkyl group having from 1 to 4 carbon atoms, an alkenyl group having from 3 to 6 carbon atoms or an aralkyl group having from 7 to 10 carbon atoms) to afford a carbamic acid ester compound of formula (VII):

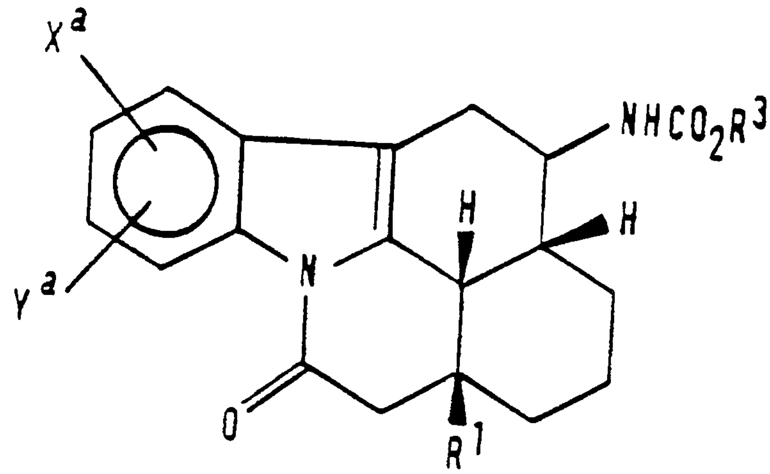

(VII)

(in which $R^1$, $R^3$, $X^a$ and $Y^a$ are as defined above);

(d) eliminating the group of formula $-CO_2R^3$ from said compound of formula (VII) to prepare the corresponding amino compound;

(e) reacting said amino compound with a compound of formula (X) or (XI):

$R^4$-Q (X)

or

Q-A-Q′ (XI)

(in which: $R^4$ represents an alkyl group having from 1 to 4 carbon atoms or an alkyl group having from 1 to 4 carbon atoms and having at least one hydroxy substituent; A represents an alkylene group having from 1 to 5 carbon atoms whose carbon chain is interrupted by 0 or 1 nitrogen, oxygen or sulphur atom; and Q and Q′ are the same or different and each represents a halogen atom); and

(f) removing the hydroxy-protecting groups;

(g) and, if desired, salifying the product.

24. A process according to Claim 23, in which said compound of formula (IV) is prepared by: reacting an indole compound of formula (II):

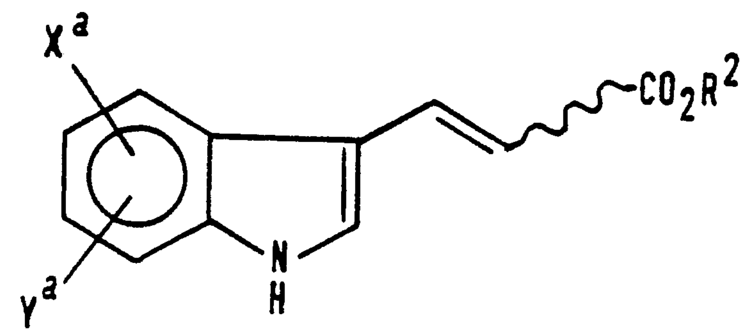

(II)

(in which $R^2$, $X^a$ and $Y^a$ are as defined in Claim 23) or a reactive derivative thereof with an optically active cyclohexenylacetic acid of formula (III):

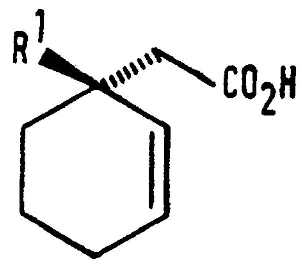

(III)

(in which $R^1$ is as defined in Claim 23) or with a reactive derivative of said acid, to afford said compound of formula (IV)

Claims for the following Contracting States: ES GR:

1. A process for preparing an optically active compound of formula (I):

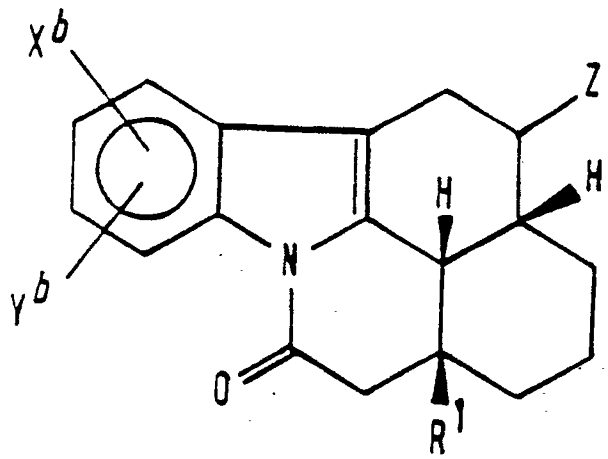

(I)

in which:
$R^1$ represents a hydrogen atom or an alkyl group having from 1 to 4 carbon atoms;
$X^b$ and $Y^b$ are the same or different and each represents a hydrogen atom or a hydroxy group; and
Z represents a group of formula $-NR^aR^b$
in which $R^a$ and $R^b$ are the same or different and each represents a hydrogen atom, an alkyl group having from 1 to 4 carbon atoms or a hydroxyalkyl group having at least one hydroxy group and having from 1 to 4 carbon atoms,
or a cyclic amino group having from 3 to 6 ring atoms, of which 1 or 2 are nitrogen atoms, 0 or 1 is an oxygen or sulphur atom and the remainder is or are carbon atoms;
and pharmaceutically acceptable salts thereof, which process comprises the steps:
(a) heating a compound of formula (IV):

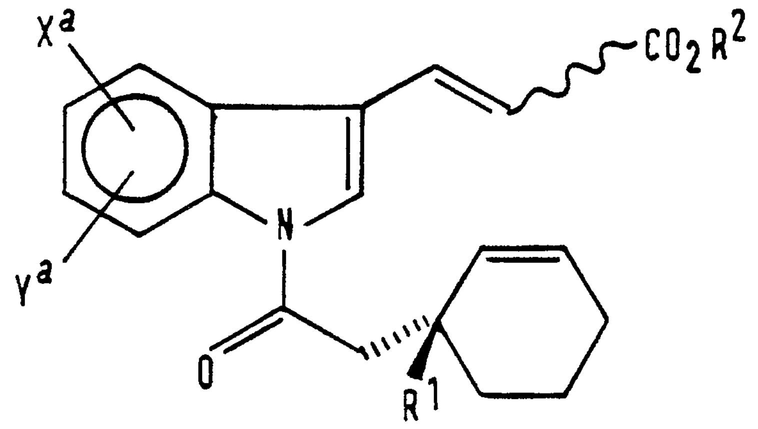

(IV)

(in which: $R^1$ is as defined above; $R^2$ represents a carboxy-protecting group; and $X^a$ and $Y^a$ are the same or different and each represents a hydrogen atom or a protected hydroxy group) to afford a compound of formula (V):

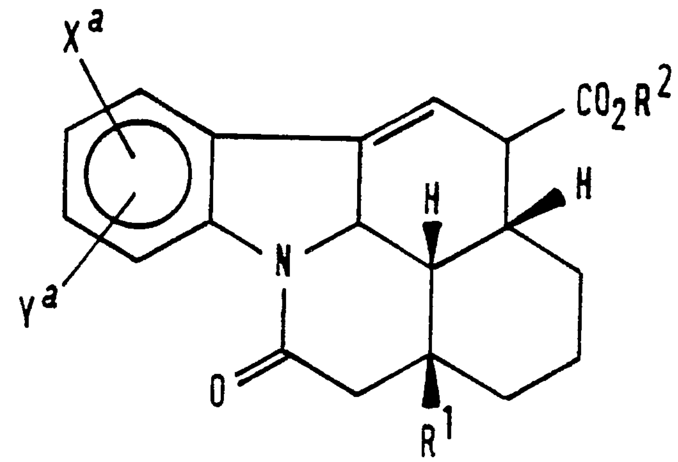

(V)

(in which $R^1$, $R^2$, $X^a$ and $Y^a$ are as defined above);
(b) isomerizing the compound of formula (V) and removing the carboxy-protecting group, to give a compound of formula (VI):

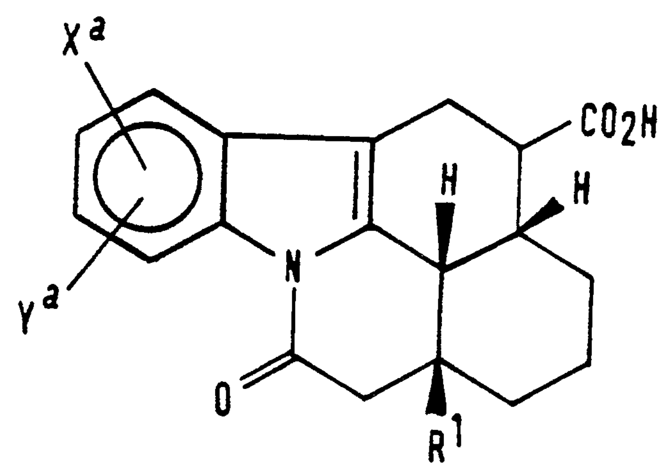

(VI)

(in which $R^1$, $X^a$ and $Y^a$ are as defined above);
(c) azidating and heating the compound of formula (VI), and then reacting it with an alcoholic compound

of formula (IX):

$R^3OH$ (IX)

(in which $R^3$ represents an alkyl group having from 1 to 4 carbon atoms, an alkenyl group having from 3 to 6 carbon atoms or an aralkyl group having from 7 to 10 carbon atoms) to afford a carbamic acid ester compound of formula (VII):

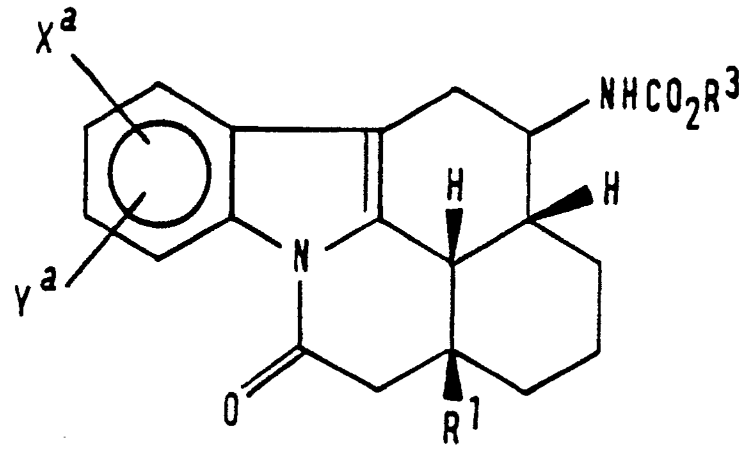

(VII)

(in which $R^1$, $R^3$, $X^a$ and $Y^a$ are as defined above);

(d) eliminating the group of formula $-CO_2R^3$ from said compound of formula (VII) to prepare the corresponding amino compound;

(e) reacting said amino compound with a compound of formula (X) or (XI):

$R^4$-Q (X)

or

Q-A-Q′ (XI)

(in which: $R^4$ represents an alkyl group having from 1 to 4 carbon atoms or an alkyl group having from 1 to 4 carbon atoms and having at least one hydroxy substituent; A represents an alkylene group having from 1 to 5 carbon atoms whose carbon chain is interrupted by 0 or 1 nitrogen, oxygen or sulphur atom; and Q and Q′ are the same or different and each represents a halogen atom); and

(f) removing the hydroxy-protecting groups;

(g) and, if desired, salifying the product.

2. A process according to Claim 1, in which said compound of formula (IV) is prepared by: reacting an indole compound of formula (II):

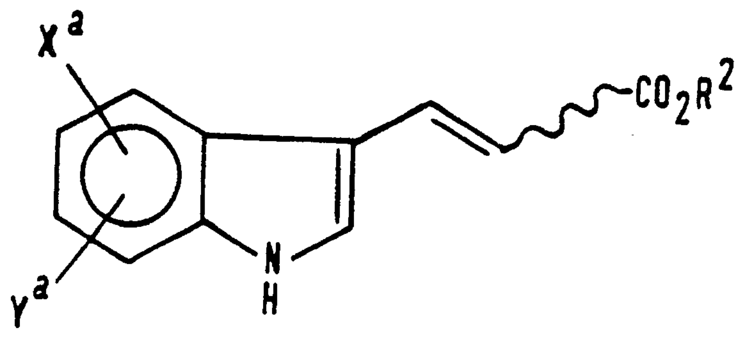

(II)

(in which $R^2$, $X^a$ and $Y^a$ are as defined in Claim 1) or a reactive derivative thereof with an optically active cyclohexenylacetic acid of formula (III):

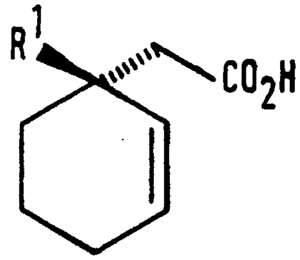

(III)

(in which $R^1$ is as defined in Claim 1) or with a reactive derivative of said acid, to afford said compound of formula (IV).

3. A process according to Claim 1 or Claim 2, in which the reagents and reaction conditions are so chosen as to prepare a compound of formula (I) or a salt thereof, in which the group represented by Z is in the R-configuration.

4. A process according to any one of Claims 1 to 3, in which the reagents and reaction conditions are so chosen as to prepare a compound of formula (I) or a salt thereof, in which $R^1$ represents a hydrogen atom, a methyl group or an ethyl group.

5. A process according to any one of Claims 1 to 3, in which the reagents and reaction conditions are so ohosen as to prepare a compound of formula (I) or a salt thereof, in which $R^1$ represents a hydrogen atom.

6. A process according to any one of Claims 1 to 5, in which the reagents and reaction conditions are so chosen as to prepare a compound of formula (I) or a salt thereof, in which one of $X^b$ and $Y^b$ represents a hydroxy group and the other represents a hydrogen atom.

7. A process according to any one of Claims 1 to 6, in which the reagents and reaction conditions are so chosen as to prepare a compound of formula (I) or a salt thereof, in which Z represents an amino, ethylamino, dimethylamino, diethylamino, pyrrolidyl or piperidyl group.

8. A process according to any one of Claims 1 to 6, in which the reagents and reaction conditions are so chosen as to prepare a compound of formula (I) or a salt thereof, in which Z represents an amino, ethylamino or dimethylamino group.

9. A process according to any one of Claims 1 to 7, in which the reagents and reaction conditions are so chosen as to prepare a compound of formula (I) or a salt thereof, in whioh $X^b$ represents a hydroxy group at the 6- or 7- position and $Y^b$ represents a hydrogen atom.

10. A process according to Claim 9, in which the reagents and reaction conditions are so chosen as to prepare a compound of formula (I) or a salt thereof, in which $X^b$ represents a hydroxy group at the 6-position.

11. A process according to any one of Claims 1 to 3, in which the reagents and reaction conditions are so chosen as to prepare a compound of formula (I) or a salt thereof, in which:

$R^1$ represents a hydrogen atom, a methyl group or an ethyl group;

one of $X^b$ and $Y^b$ represents a hydroxy group and the other represents a hydrogen atom; and

Z represents an amino, ethylamino, dimethylamino, diethylamino, pyrrolidyl or piperidyl group.

12. A process according to any one of Claims 1 to 3, in whioh the reagents and reaction conditions are so chosen as to prepare a compound of formula (I) or a salt thereof, in which:

$R^1$ represents a hydrogen atom;

one of $X^b$ and $Y^b$ represents a hydroxy group and the other represents a hydrogen atom; and

Z represents an amino, ethylamino or dimethylamino group.

13. A process according to Claim 12, in which the reagents and reaction conditions are so chosen as to prepare a compound of formula (I) or a salt thereof, in which $X^b$ represents a hydroxy group at the 6-position.

14. A process according to Claim 1 or Claim 2, in which the reagents and reaction conditions are so chosen as to prepare a compound of formula (I) or a salt thereof, in which said compound is selected from:

(3a R ,4 R ,12a R ,12b S ) 4-amino-2,3,3a,4,5,12,12a,12b-octahydro-6-hydroxyindolo[3,2,1,-ij]benz[de]-quinolin-11(1 H )-one;

(3a R ,4 R ,12a R ,12b S ) 4-amino-2,3,3a,4,5,12,12a,12b-octahydro-6-hydroxy-12a-methylindolo[3,2,1,-ij]-benz[de]quinolin-11(1 H )-one;

(3a R ,4 R ,12a R ,12b S ) 4-amino-12a-ethyl-2,3,3a,4,5,12,12a,12b-octahydro-6-hydroxyindolo[3, 2,1, -ij]-benz[de]quinolin-11(1 H )-one;

(3a R , 4 R , 12a R , 12b S ) 4-(dimethylamino)-2,3,3a,4,5,12,12a,12b-octahydro-6-hydroxyindolo[3,2,1,-ij]-benz[de]quinolin-11(1 H )-one;

(3a R , 4 R ,12a R ,12b S ) 4-ethylamino-2,3,3a,4,5,12,12a,12b-octahydro-6-hydroxyindolo[3,2,1,-ij]benz[de]-quinolin-11(1 H )-one;
(3a R , 4 R ,12a R ,12b S ) 2,3,3a,4,5,12,12a,12b-octahydro-6-hydroxy-4-pyrrolidin-1-ylindolo-[3, 2, 1, -ij]-benz[de]quinolin-11(1 H )-one; and
(3a R ,4 R ,12a R ,12b S ) 2,3,3a,4,5,12,12a,12b-octahydro-6- hydroxy-4-bis(2-hydroxyethyl)aminoindolo-[3,2,1-ij]benz[de]quinolin-11(1 H )-one;
and pharmaceutically acceptable salts thereof.

15. The use of at least one optically active compound of formula (I) or a pharmaceutically acceptable salt thereof, as defined in any one of Claims 1 to 14, for the manufacture of a medicament for the treatment or prophylaxis of arrhythmia in a mammal

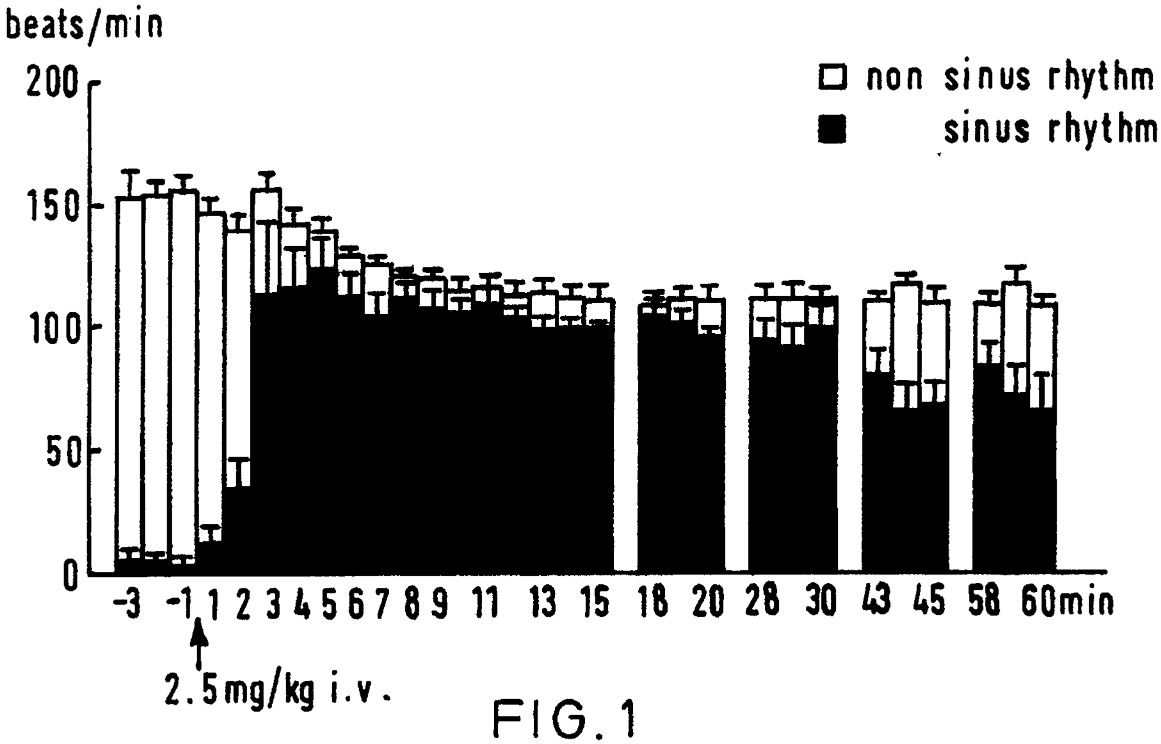


FIG. 1
Effect of the compound in Example 1 (N=5)

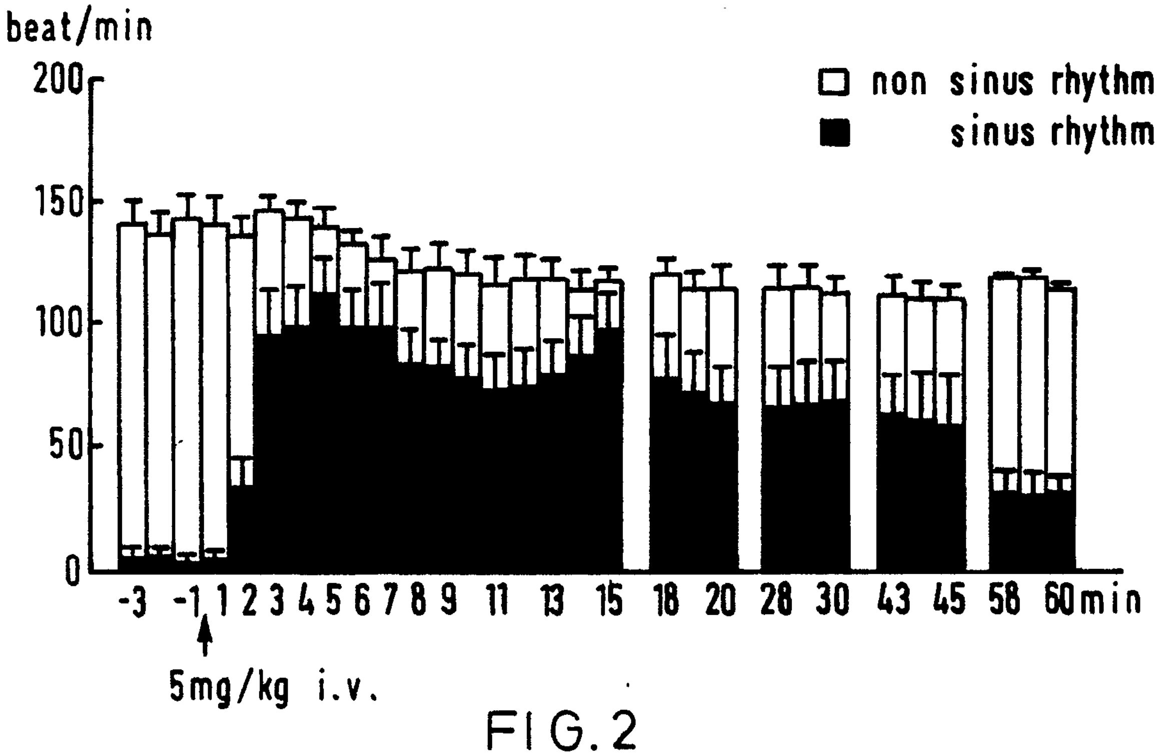


FIG. 2
Effect of the racemate of the compound in Example 1 (N=3)